(19) Europäisches Patentamt
European Patent Office
Office européen des brevets

(11) **EP 2 278 331 A1**

(12) **EUROPEAN PATENT APPLICATION**
published in accordance with Art. 153(4) EPC

(43) Date of publication:
**26.01.2011 Bulletin 2011/04**

(21) Application number: **09738865.6**

(22) Date of filing: **30.04.2009**

(51) Int Cl.:
***G01N 33/49*** *(2006.01)*

(86) International application number:
**PCT/JP2009/058489**

(87) International publication number:
**WO 2009/133928 (05.11.2009 Gazette 2009/45)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **02.05.2008 JP 2008120248**

(71) Applicant: **ARKRAY, Inc.**
**Kyoto-shi, Kyoto 601-8045 (JP)**

(72) Inventor: **The designation of the inventor has not yet been filed**

(74) Representative: **Golding, Louise Ann et al**
**Dehns**
**St Bride's House**
**10 Salisbury Square**
**London**
**EC4Y 8JD (GB)**

(54) **LEUKOCYTE ANALYSIS METHOD AND ANALYSIS REAGENT FOR USE IN THE METHOD**

(57) The present invention provides a method for analyzing leukocytes, by which the leukocytes can be classified and measured stably with high accuracy even when a dilution ratio of a sample containing the leukocytes is low or a flow velocity during the analysis is slow, and an analysis reagent used for the analysis method. The analysis method of the present invention includes the steps of mixing a sample containing leukocytes and erythrocytes and an analysis reagent containing a surfactant that reacts with leukocytes; and measuring the leukocytes by passing a mixed solution of the sample and the analysis reagent through a fine through-hole, measuring a signal detected when the mixed solution passes through the fine through-hole, and classifying and counting the leukocytes in the sample. The analysis reagent further contains a nonionic surfactant, and the nonionic surfactant has a sugar residue as a hydrophilic region and an aliphatic chain as a hydrophobic region.

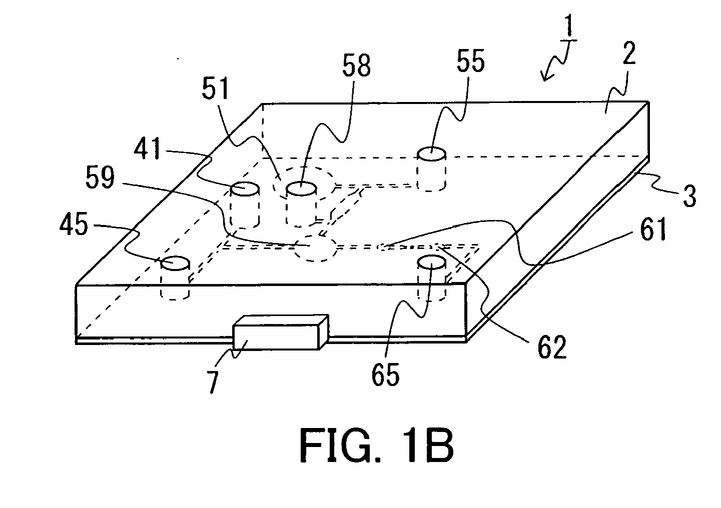

EP 2 278 331 A1

FIG. 1A

FIG. 1B

**Description**

Technical Field

The present invention relates to a leukocyte analysis method and an analysis reagent for use in the method.

Background Art

In the medical diagnoses, blood analyses are often carried out. The analytes of the blood analysis include a leukocyte, an erythrocyte, a concentration of hemoglobin, a hematocrit value, and the like. From the numbers, concentrations, proportions, and the like of these objects to be analyzed, it is possible to diagnose blood diseases such as plethora (erythrocytosis) and anemia and diseases such as infectious diseases. Among them, the number or proportion of leukocytes is an important indicator to diagnose disorders such as infectious diseases. Therefore, in order to improve the accuracy of classifying and counting leukocytes in blood, various analysis methods are developed. As the leukocyte analysis method, a method in which a lyse reagent is caused to react with leukocytes and erythrocytes (for example, Patent Citation 1) and the like are proposed.

These days, in the analyses and the like of biological samples in medical diagnoses, a micro total analysis system (hereinafter referred to as a "μTAS") is considered. The μTAS is a chemical analysis system in which a micro flow path, a pump, a valve, a sensor, and the like are assembled together on a substrate such as silicon and has advantages in that the amount of a sample to be used can be reduced, the cartridge for measurement can be a disposable cartridge, the device can be downsized, and the like. The development of an analysis method that uses the μTAS is desired because the leukocyte is an analysis item of the mass screening. However, since the μ TAS is a micro system, a dilution ratio of a sample becomes low, and a flow velocity during an analysis becomes slow. In the conventional leukocyte analysis method, when a dilution ratio of a sample is low, there is a risk that erythrocytes in the sample affect a reaction of leukocytes with an analysis reagent, and there is a case where a measurement result of leukocytes changes depending on an erythrocyte content (hematocrit value and the like). Therefore, stable analysis cannot be carried out. Further, when the flow velocity during the analysis is slow, the number of leukocytes that can be measured becomes small, resulting in low analysis accuracy. Therefore, to increase the accuracy of the analysis, it is required to lengthen the measurement time and increase the total flow rate. However, in the conventional leukocyte analysis method, as mentioned above, when the measuring time is set to be long, the reaction of leukocytes with the analysis reagent progresses with the measurement time, so that stable analysis cannot be carried out.

Citation List

Patent Literature

Patent Literature 1: JP No. 3143064

Summary of Invention

Technical Problem

Hence, the present invention is intended to provide a method for analyzing leukocytes, by which leukocytes can be classified and measured stably even in the case where, for example, a dilution ratio of a sample containing leukocytes and erythrocytes is low, or a flow velocity during the analysis is slow, and an analysis reagent used for the analysis method. Solution to Problem

In order to achieve the aforementioned object, the analysis method of the present invention is a method for analyzing a leukocyte, including the steps of: mixing a sample containing a leukocyte and an erythrocyte and an analysis reagent containing a surfactant that reacts with leukocytes; and measuring the leukocyte by passing a mixed solution of the sample and the analysis reagent through a fine through-hole, measuring a signal detected when the mixed solution passes through the fine through-hole, and classifying and counting the leukocyte in the sample, wherein the analysis reagent further contains a nonionic surfactant, and the nonionic surfactant has a sugar residue as a hydrophilic region and an aliphatic chain as a hydrophobic region.

The analysis reagent of the present invention is an analysis reagent used for the analysis method of the present invention, containing a surfactant that reacts with leukocytes, wherein the analysis reagent further contains a nonionic surfactant, and the nonionic surfactant has a sugar residue as a hydrophilic region and an aliphatic chain as a hydrophobic region. Advantageous Effects of Invention

According to the present invention, even in the case where, for example, a dilution ratio of a sample containing leukocytes and erythrocytes is low, an influence of the erythrocytes on a reaction of the leukocytes with an analysis reagent is suppressed and it is possible to carry out a stable analysis of the leukocytes. Further, according to the present invention, a reaction of the analysis reagent with leukocytes proceeds sluggishly even in the case where, for example, a flow velocity during the analysis is slow. Thus, a measurement time can be set longer, a flow rate that is sufficient to carry out the analysis can be obtained, and stable analysis can be carried out. Furthermore, since the present invention is applicable to the μTAS, it is possible to reduce the amount of the sample to be used, to use a disposable cartridge, and to downsize an analyzing device, for example.

Brief Description of Drawings

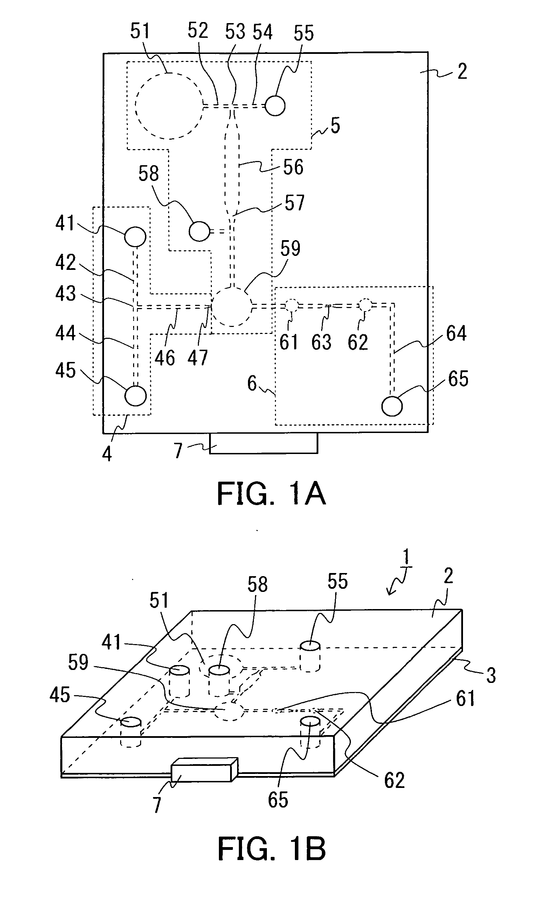

[FIG. 1] FIG. 1A is a plan view showing an embodiment of a cartridge used in the analysis method of the present invention. FIG. 1B is a perspective view of the cartridge shown in FIG. 1A.

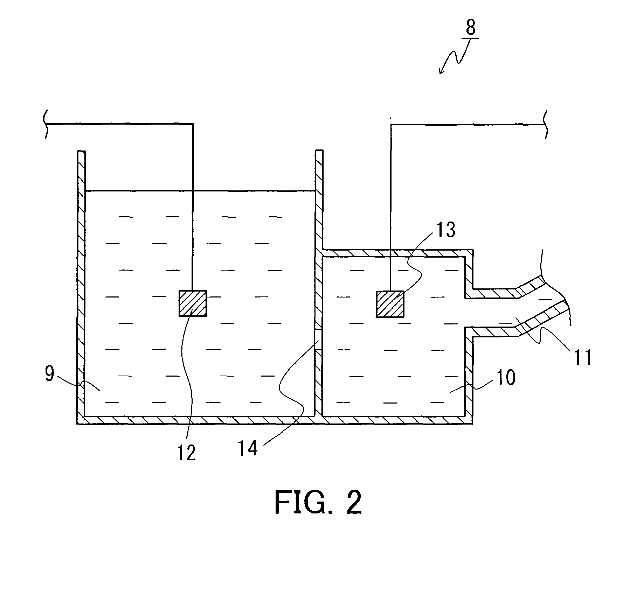

[FIG. 2] FIG. 2 is a cross-sectional view showing an embodiment of a leukocyte analysis device used in the analysis method of the present invention.

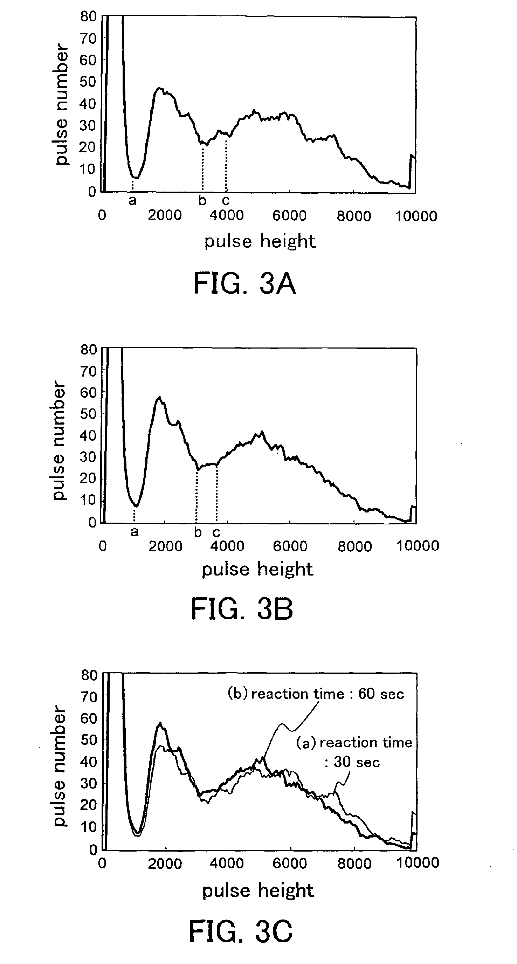

[FIG. 3] FIG. 3A is a histogram showing a particle size distribution of leukocytes in the case where the reaction time was 30 seconds in an example of the preset invention. FIG. 3B is a histogram showing a particle size distribution of leukocytes in the case where the reaction time was 60 seconds in the example of the present invention. FIG. 3C is histograms showing particle size distributions of leukocytes in the case where the reaction times were 30 and 60 seconds, respectively, in the example of the present invention.

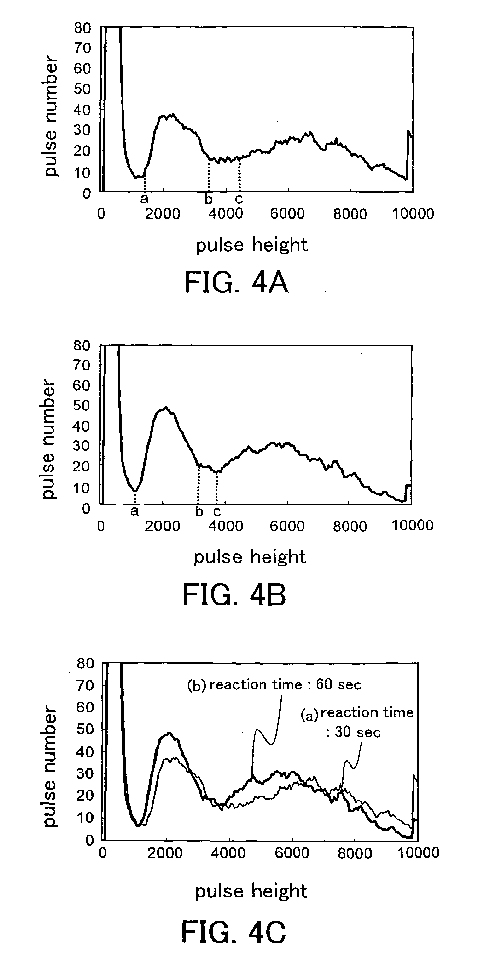

[FIG. 4] FIG. 4A is a histogram showing a particle size distribution of leukocytes in the case where the reaction time was 30 seconds in another example of the present invention. FIG. 4B is a histogram showing a particle size distribution of leukocytes in the case where the reaction time was 60 seconds in the another example of the present invention. FIG. 4C is histograms showing particle size distributions of leukocytes in the case where the reaction times were 30 and 60 seconds, respectively, in the another example of the present invention.

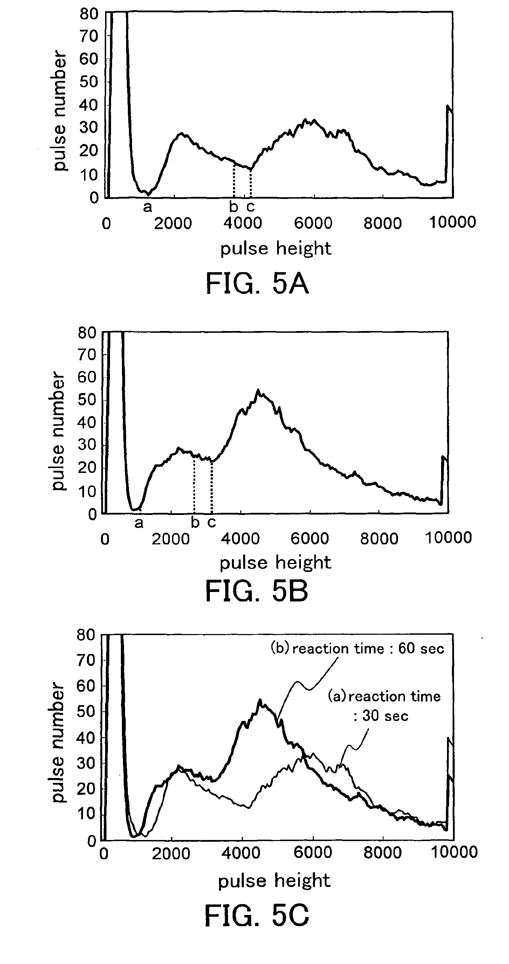

[FIG. 5] FIG. 5A is a histogram showing a particle size distribution of leukocytes in the case where the reaction time was 30 seconds in a comparative example. FIG. 5B is a histogram showing a particle size distribution of leukocytes in the case where the reaction time was 60 seconds in the comparative example. FIG. 5C is histograms showing particle size distributions of leukocytes in the case where the reaction times were 30 and 60 seconds, respectively, in the comparative example.

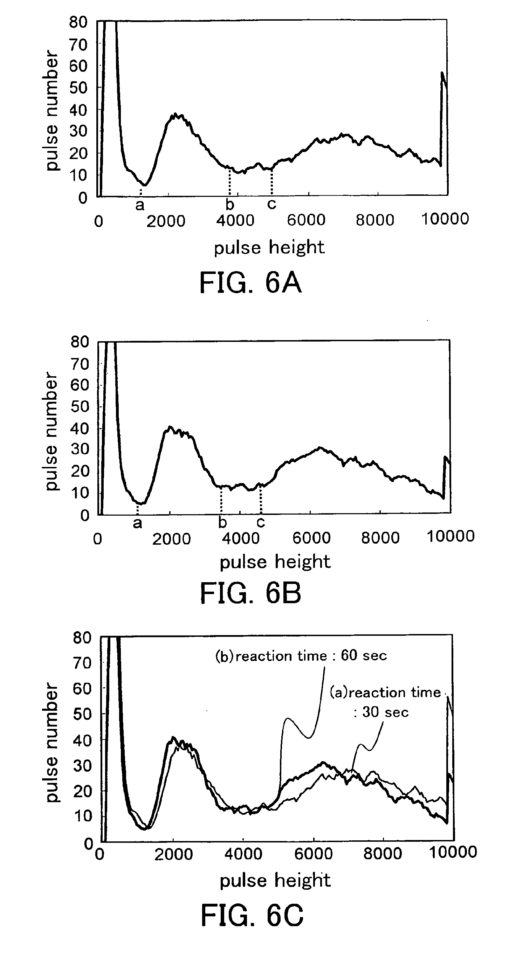

[FIG. 6] FIG. 6A is a histogram showing a particle size distribution of leukocytes in the case where the reaction time was 30 seconds in another comparative example. FIG. 6B is a histogram showing a particle size distribution of leukocytes in the case where the reaction time was 60 seconds in the another comparative example. FIG. 6C is histograms showing particle size distributions of leukocytes in the case where the reaction times were 30 and 60 seconds, respectively, in the another comparative example.

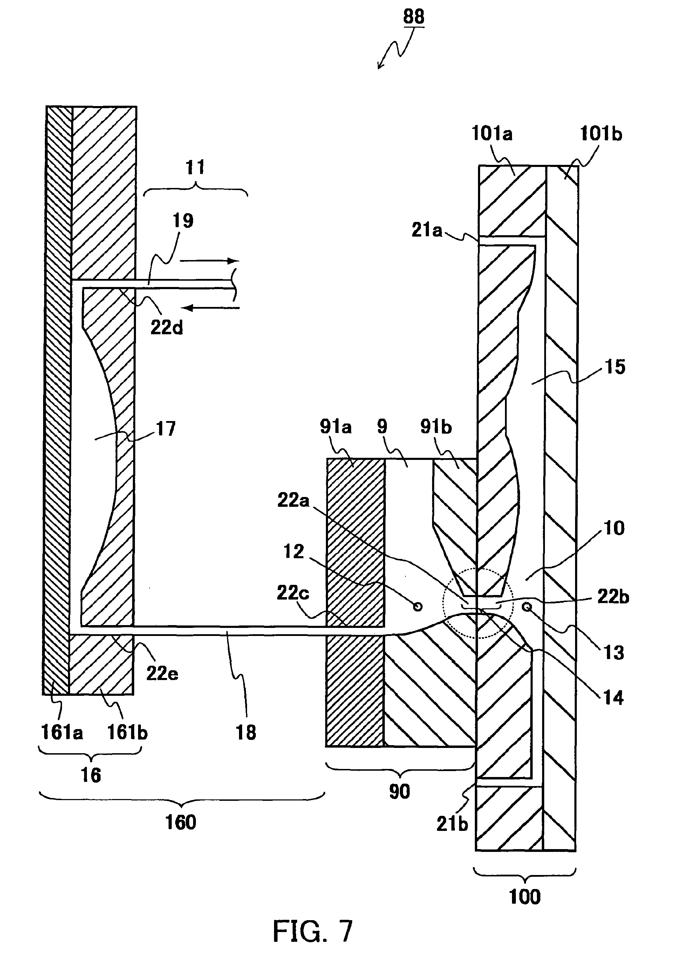

[FIG. 7] FIG. 7 is a cross-sectional view showing another embodiment of the leukocyte analysis device used in the analysis method of the present invention.

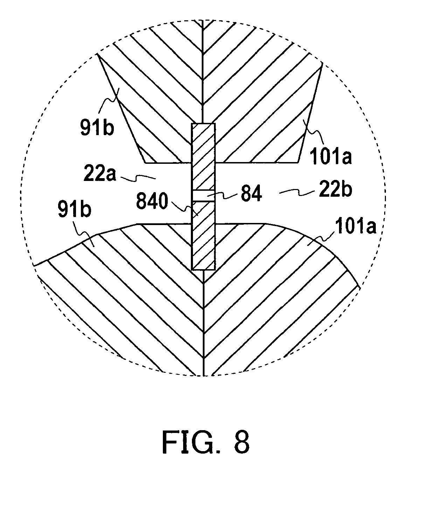

[FIG. 8] FIG. 8 is a cross-sectional view showing another example of a fine through-hole part of the leukocyte analysis device used in the analysis method of the present invention.

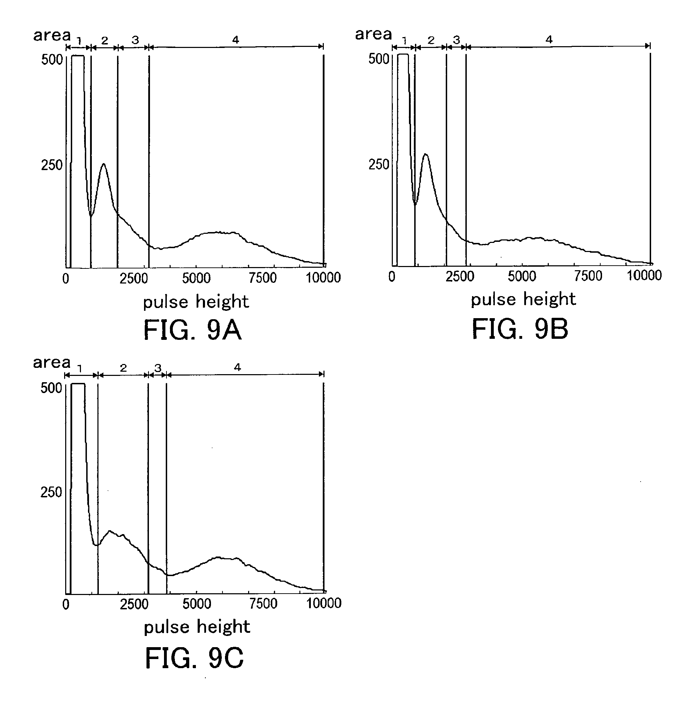

[FIG. 9] FIGs. 9A to 9C are histograms showing particle size distributions of leukocytes in the analyses using the various analysis reagents of comparative examples, respectively. FIG. 9A is a histogram showing an analysis result in the case where a basic reagent was used. FIG. 9B is a histogram showing an analysis result in the case where an analysis reagent to which lauryl trimethyl ammonium chloride was added was used. FIG. 9C is a histogram showing an analysis result in the case where an analysis reagent to which polyoxyethylene lauryl ether was added was used.

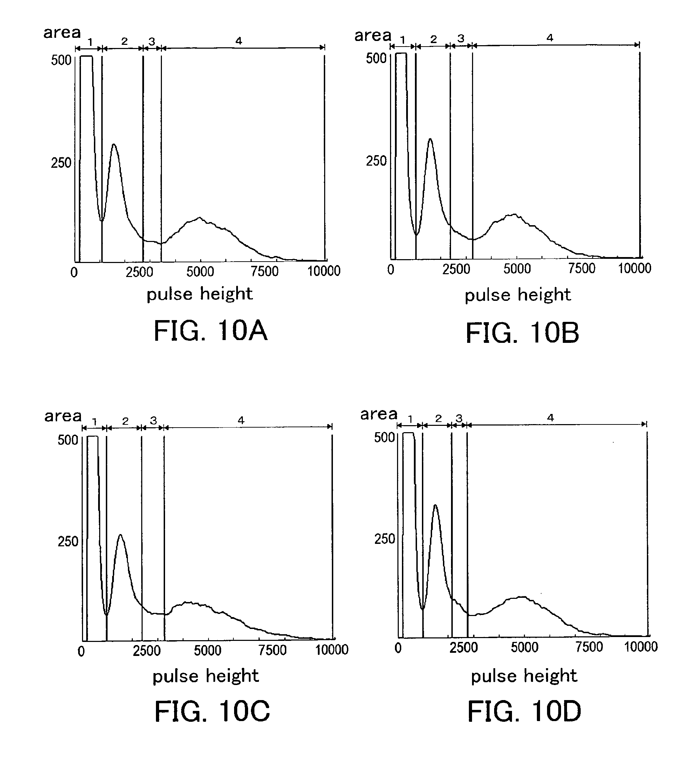

[FIG. 10] FIGs. 10A to 10D are histograms showing particle size distributions of leukocytes in the analyses using the various analysis reagents of the present invention, respectively. FIG. 10A is a histogram showing an analysis result in the case where an analysis reagent to which sucrose laurate was added was used. FIG. 10B is a histogram showing an analysis result in the case where an analysis reagent to which sucrose monolaurate was added was used. FIG. 10C is a histogram showing an analysis result in the case where an analysis reagent to which sucrose monocaprate was added was used. FIG. 10D is a histogram showing an analysis result in the case where an analysis reagent to which dodecyl maltoside was added was used.

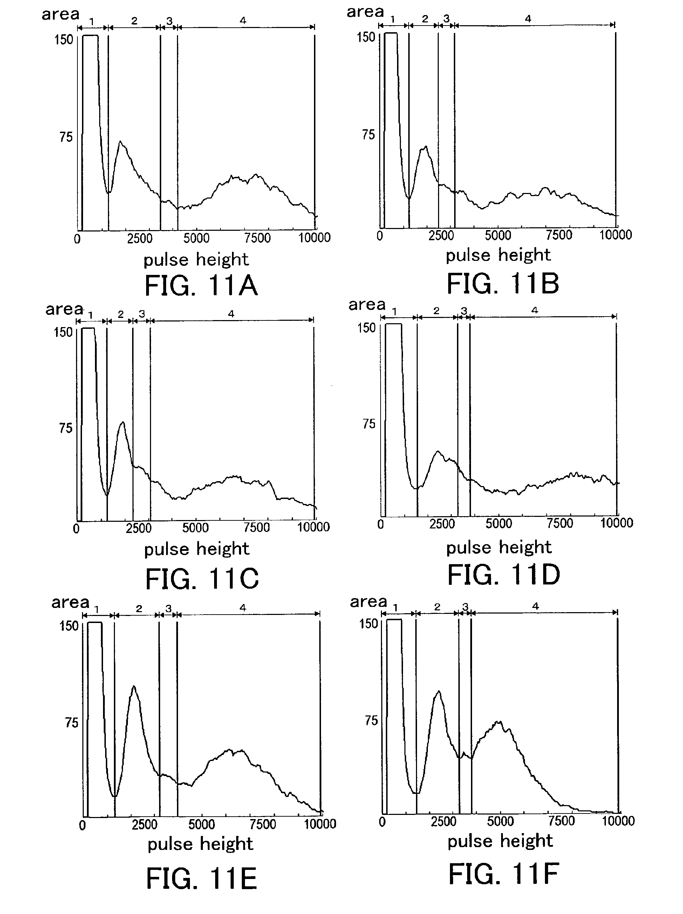

[FIG. 11] FIGs 11A to 11F are histograms showing particle size distributions of leukocytes in the analyses using the various analysis reagents having predetermined concentrations of sucrose laurate, respectively. FIG. 11A is a histogram showing an analysis result in the case where an analysis reagent to which 0 w/v% sucrose laurate was added (sucrose laurate was not added) was used. FIG. 11B is a histogram showing an analysis result in the case where an analysis reagent to which 0.0125 w/v% sucrose laurate was added was used. FIG. 11C is a histogram showing an analysis result in the case where an analysis reagent to which 0.025 w/v% sucrose laurate was added was used. FIG. 11D is a histogram showing an analysis result in the case where an analysis reagent to which 0.05 w/v% sucrose laurate was added was used. FIG. 11E is a histogram showing an analysis result in the case where an analysis reagent to which 0.1 w/v% sucrose laurate was added was used. FIG. 11F is a histogram showing an analysis result in the case where an analysis reagent to which 0.2 w/v% sucrose laurate was added was used.

Description of Embodiments

In the analysis method of the present invention, the sugar residue in the nonionic surfactant preferably is a disaccharide residue.

In the analysis method of the present invention, the aliphatic chain in the nonionic surfactant preferably is a fatty acid residue or an alkyl group.

In the analysis method of the present invention, the nonionic surfactant preferably is at least one selected from the group consisting of sucrose monolaurate, sucrose laurate, sucrose monocaprate, and dodecyl maltoside.

In the analysis method of the present invention, the nonionic surfactant preferably is sucrose monolaurate or sucrose laurate.

In the analysis method of the present invention, a concentration of the nonionic surfactant in the mixed solution of the sample and the analysis reagent preferably is in a range of 0.001 to 5 w/v%.

In the analysis method of the present invention, the surfactant that reacts with leukocytes preferably is a quaternary ammonium salt.

In the analysis method of the present invention, a concentration of the surfactant that reacts with leukocytes in the mixed solution of the sample and the analysis reagent preferably is in a range of 0.01 to 5 w/v%.

In the analysis method of the present invention, the analysis reagent preferably further contains a surfactant that reacts with erythrocytes.

In the analysis method of the present invention, the surfactant that reacts with erythrocytes preferably is a saponin.

In the analysis method of the present invention, a concentration of the saponin in the mixed solution of the sample and the analysis reagent preferably is in a range of 0.05 to 5 w/v%.

In the mixing step of the analysis method of the present invention, a mixed volume ratio (X:Y) of the sample (X) to the analysis reagent (Y) preferably is in a range of 1:0.4 to 1:99.

In the measuring step of the analysis method of the present invention, the leukocyte preferably is classified into three types according to its volume.

In the measuring step of the analysis method of the present invention, preferably, a cartridge having the fine through-hole is used, and the mixed solution is passed through the fine through-hole in the cartridge.

In the analysis method of the present invention, the cartridge preferably is a micro total analysis system.

In the analysis reagent of the present invention, the sugar residue preferably is a disaccharide.

In the analysis reagent of the present invention, the aliphatic chain preferably is a fatty acid residue or an alkyl group.

In the analysis reagent of the present invention, the nonionic surfactant preferably is at least one selected from the group consisting of sucrose monolaurate, sucrose laurate, sucrose monocaprate, and dodecyl maltoside.

In the analysis reagent of the present invention, the surfactant that reacts with leukocytes preferably is a quaternary ammonium salt.

The analysis reagent of the present invention preferably further contains a surfactant that reacts with erythrocytes.

In the analysis reagent of the present invention, the surfactant that reacts with erythrocytes preferably is a saponin.

Next, the present invention will be described with reference to examples.

<Analysis method>

As mentioned above, the analysis method of the present invention is a method for analyzing a leukocyte, including the steps of mixing a sample containing a leukocyte and an erythrocyte and an analysis reagent containing a surfactant that reacts with leukocytes; and measuring the leukocyte by passing a mixed solution of the sample and the analysis reagent through a fine through-hole, measuring a signal detected when the mixed solution passes through the fine through-hole, and classifying and counting the leukocyte in the sample, wherein the analysis reagent further contains a nonionic surfactant, and the nonionic surfactant has a sugar residue as a hydrophilic region and an aliphatic chain as a hydrophobic region. It is to be noted that the analysis reagent used in the analysis method of the present invention corresponds to an analysis reagent of the present invention.

The present invention permits carrying out the analysis that is less susceptible to an influence of erythrocyte content (hematocrit value and the like) and is stable. Further, according to the present invention, separation accuracy of blood components (noise) detected as pulses smaller than those of lymphocytes and lymphocytes can be improved, and also, separation accuracy of the lymphocytes and the granulocytes can be improved. Therefore, according to the present invention, it is possible to improve the analysis accuracy.

Mixing step

In the mixing step, the sample and the analysis reagent are mixed to cause the leukocytes and the erythrocytes in the sample to react with the analysis reagent. The reaction of the analysis reagent with the leukocytes can be, for example: obtaining bare nuclei of the leukocytes by dissolving cell membranes of leukocytes; shrinking or swelling leukocyte by a change in osmotic pressure: or the like. In the present invention, a reaction of the analysis reagent with the erythrocytes can be, for example, hemolysis, dissolution of erythrocyte membranes, or the like.

The sample containing leukocytes and erythrocytes is not particularly limited, and examples thereof include a blood sample, and a treated blood sample. Examples of the blood sample include whole blood and hemocytes. The treatment of the blood sample is not particularly limited, and can be, for example, a dilution treatment. The dilution treatment is not particularly limited, and as a diluent, a physiological saline solution, a buffer, or the like can be used, for example. The dilution ratio is not particularly limited, and the blood sample is diluted, for example, by a factor in the range of 1 to 500, preferably by a factor in the range of 5 to 400, and more preferably by a factor in the range of 200 to 300. For example, in the case where whole blood is diluted, when the diluted sample and the analysis reagent are mixed, the whole blood is ultimately diluted preferably by a factor in the range of 5 to 1000, more preferably by a factor in the range of 15 to 400. In this case, a concentration range of each component of the analysis reagent in the mixed solution obtained by mixing the diluted sample and the analysis reagent preferably is a range that will be mentioned later.

The buffer is not particularly limited, and examples thereof include an ADA (N-(2-acetamide)iminodiacetic acid) buffer, an MES (2-morpholinoethanesulfonic acid) buffer, a Bis-Tris (bis-(2-hydroxyethyl)imino-tris-(hydroxymethylmethane) buffer, a PIPES (piperazine-1,4-bis(2-ethanesulfonic acid)) buffer, an ACES (N-(2-acetamido)-2-aminoethanesulfonic acid) buffer, a MOPSO (2-hydroxy-3-morpholinopropanesulfonic acid) buffer, a BES (N,N-bis(2-hydroxyethyl)-2-aminoethanesulfonic acid) buffer, a HEPES (2-[4-(2-hydroxyethyl)-1-piperazinyl]ethanesulfonic acid) buffer, and a phosphate buffer. The pH of the buffer is, for example, in the range of 2 to 12, preferably in the range of 5 to 7.5.

The species of animals from which the sample is collected are not particularly limited, and examples thereof include mammals such as humans, cattle, horses, dogs, and cats.

In the present invention, the analysis reagent contains the leukocyte-reactive surfactant and the nonionic surfactant.

The leukocyte-reactive surfactant is not particularly limited, and examples thereof include quaternary ammonium salts, a saponin, a polyoxyethylene nonionic surfactant, and a polyoxyethylene anionic surfactant. The leukocyte-reactive surfactant preferably is a quaternary ammonium salt. The leukocyte-reactive surfactants may be used alone or in a combination of two or more of them.

The quaternary ammonium salt is not particularly limited, and examples thereof include lauryl trimethyl ammonium chloride, cetyl trimethyl ammonium chloride, tetratrimethyl ammonium chloride, stearyl trimethyl ammonium chloride, behenyl trimethyl ammonium chloride, coco benzyl dimethyl ammonium chloride, didecyl dimethyl ammonium chloride, and myristyl trimethyl ammonium bromide. The quaternary ammonium salt preferably is lauryl trimethyl ammonium chloride, cetyl trimethyl ammonium chloride, or tetratrimethyl ammonium chloride. The quaternary ammonium salts may be used alone or in a combination of two or more of them.

The concentration of the leukocyte-reactive surfactant in the mixed solution of the sample and the analysis reagent is not particularly limited, and can be, for example, set as appropriate depending on the type of the surfactant. The concentration is, for example, in the range of 0.01 to 5 w/v%, preferably in the range of 0.075 to 1 w/v%. In the case where the leukocyte-reactive surfactant is the quaternary ammonium salt, the concentration of the quaternary ammonium salt in the mixed solution of the sample and the analysis reagent is not particularly limited, and is, for example, in the above-mentioned concentration range.

In the present invention, the nonionic surfactant has, as described above, a sugar residue as a hydrophilic region and an aliphatic chain as a hydrophobic region.

The sugar residue is not particularly limited, and can be, for example, a sugar residue such as a monosaccharide, a disaccharide, or an oligosaccharide. The number of monosaccharides in the sugar residue is not particularly limited, and may be, for example, in the range of 1 to 20. The sugar residue of the monosaccharide is not particularly limited, and examples thereof include a glucose residue, a galactose residue, a mannose residue, a thioglucose residue, an arabinose residue, a xylose residue, a glucuronic acid residue, and a glucosamine residue. The sugar residue of the disaccharide is not particularly limited, and examples thereof include a sucrose residue, a lactose residue, a maltose residue, and a thiomaltose residue. The sugar residue of the disaccharide preferably is a sucrose residue.

The aliphatic chain is not particularly limited, and examples thereof include a fatty acid residue and an alkyl group. The fatty acid residue is not particularly limited, and may be, for example, a saturated fatty acid residue or an unsaturated fatty acid residue. Examples of the fatty acid residue include a straight-chain fatty acid residue, a branched-chain fatty acid residue, and a cyclic fatty acid residue, and the fatty acid residue is not particularly limited. The carbon number of the fatty acid residues is not particularly limited, and is, for example, in the range of 4 to 28, preferably in the range of 10 to 22. The saturated fatty acid residue is not particularly limited, and examples thereof include a capric acid residue, a lauric acid residue, a myristic acid residue, a pentadecylic acid residue, a palmitic acid residue, a stearic acid residue, an arachidic acid residue, and a behenic acid residue. The saturated fatty acid residue preferably is a capric acid residue or a lauric acid residue. These residues preferably are, for example, acyl groups such as a decanoyl group, a dodecanoyl group, a tetradecanoyl group, a pentadecanoyl group, a hexadecanoyl group, an octadecanoyl group, an icosanoyl group, and a docosanoyl group. The unsaturated fatty acid residue is not particularly limited, and examples thereof include an oleic acid residue and a linoleic acid residue. These residues preferably are, for example, acyl groups such as a cis-9-octadecenoyl group and a cis, cis-9, 12-octadecadienoyl group.

The alkyl group is not particularly limited, and may be, for example, a straight-chain alkyl group or a branched-chain alkyl group. The carbon number of the alkyl group is not particularly limited and may be, for example, in the range of 1 to 18. Specific examples of the alkyl group are not particularly limited and include a methyl group, an ethyl group, an n-propyl group, an isopropyl group, a n-butyl group, an isobutyl group, a sec-butyl group, a tert-buthyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an icosyl group.

Specific examples of the nonionic surfactant are not particularly limited and include a sucrose fatty acid ester, an alkyl glucoside, and an alkyl oligosaccharide.

The sucrose fatty acid ester is not particularly limited, and examples thereof include sucrose caprate, sucrose laurate, sucrose myristate, sucrose palmitate, sucrose stearate, sucrose behenate, sucrose oleate, and sucrose linoleate. The sucrose fatty acid ester preferably is sucrose caprate or sucrose laurate. Examples of the sucrose fatty acid ester include monoesters, diesters, and triesters and are not particularly limited. The sucrose fatty acid monoester is not particularly limited, and examples thereof include sucrose monocaprate, sucrose monolaurate, sucrose monomyristate, sucrose monopalmitate, sucrose monostearate, sucrose monobehenate, sucrose monooleate, and sucrose monolinoleate. The sucrose fatty acid monoester preferably is sucrose monocaprate or sucrose monolaurate. The sucrose fatty acid diester is not particularly limited, and examples thereof include sucrose dicaprate, sucrose dilaurate, sucrose dimyristate, sucrose dipalmitate, sucrose distearate, sucrose dibehenate, sucrose dioleate, and sucrose dilinoleate. The sucrose fatty acid diester preferably is sucrose dicaprate or sucrose dilaurate. The sucrose fatty acid triester is not particularly limited, and examples thereof include sucrose tricaprate, sucrose trilaurate, sucrose trimyristate, sucrose tripalmitate, sucrose tristearate, sucrose tribehenate, sucrose trioleate, and sucrose trilinoleate. The sucrose fatty acid triester preferably is sucrose tricaprate or sucrose trilaurate.

The sucrose fatty acid ester may be, for example, any of a monoester, a diester, and a trimester or a mixture thereof. The sucrose fatty acid ester preferably contains the monoester, for example. The proportion of the sucrose fatty acid monoester is not particularly limited, and is, for example, in the range of 50% to 100% by volume, preferably in the range of 70% to 100% by volume.

The alkyl glucoside is not particularly limited, and examples thereof include n-octyl-B-D-glucoside, n-dodecyl-B-maltoside, n-decyl-B-maltoside, n-octyl-B-D-maltoside, 3-oxatridecyl-$\alpha$-D-mannoside, n-heptyl-B-thioglucoside, n-nonyl-B-D-thiomaltoside, and n-octyl-B-D-thioglucoside.

The nonionic surfactants may be, for example, used alone or in a combination of two or more of them.

The concentration of the nonionic surfactant in the mixed solution of the sample and the analysis reagent is not particularly limited, and is, for example, in the range of 0.001 to 5 w/v%, preferably in the range of 0.001 to 1 w/v%, and more preferably in the range of 0.01 to 1 w/v%.

The analysis reagent preferably further contains a surfactant that reacts with erythrocytes (hereinafter, referred to as an erythrocyte-reactive surfactant) in addition to the leukocyte-reactive surfactant and the nonionic surfactant, as mentioned above.

In the present invention, the erythrocyte-reactive surfactant is not particularly limited, and may be, for example, a chemically synthesized surfactant or a surfactant derived from a natural product. The chemically synthesized surfactant is not particularly limited, and examples thereof include a cationic surfactant, an anionic surfactant, and an amphoteric surfactant. The cationic surfactant is not particularly limited, and examples thereof include dodecylpyridinium bromide and cetylpyridinium chloride. The anionic surfactant is not particularly limited, and examples thereof include sodium dodecylsulfate and sodium tetradecyl sulfonate. The amphoteric surfactant is not particularly limited, and examples thereof include CHAPS (3-[(3-cholamidopropyl)dimethylammonio]-1-propanesulfonate) and dodecyl-N-betaine. The surfactant derived from a natural product is also not particularly limited, and examples thereof include a saponin, a casein, and a lecithin. Among the surfactants, the saponin is preferable as the erythrocyte-reactive surfactant. It is to be noted that the saponin may serve as both the leukocyte-reactive surfactant and the erythrocyte-reactive surfactant.

The concentration of the erythrocyte-reactive surfactant in the mixed solution of the sample and the analysis reagent is not particularly limited, and can be, for example, set as appropriate depending on the surfactant to be used. The concentration of the same is not particularly limited, and is, for example, in the range of 0.05 to 5 w/v%, preferably in the range of 0.1 to 0.5 w/v%. In the case of using the saponin, the concentration of the saponin in the mixed solution of the sample and the analysis reagent is not particularly limited, and is, for example, in the above-mentioned concentration range.

In the present invention, a composition of the analysis reagent other than the surfactant is not particularly limited, and for example, the analysis reagent may contain a buffer, an additive, and the like. The buffer is not particularly limited, and can be, for example, any of those mentioned above. The pH of the buffer is not particularly limited and is, for example, in the above-mentioned pH range.

The additive is not particularly limited, and examples thereof include a reaggregation inhibitor and an additive for measuring hemoglobin.

The reaggregation inhibitor is, for example, a solution for inhibiting reaggregation of components of a blood sample. The reaggregation inhibitor is not particularly limited, and examples thereof include sodium citrate, heparin, EDTA (ethylenediaminetetraacetic acid), sodium fluoride, and ACD (acid citrate-dextrose solution).

The concentration of the raggregation inhibitor in the mixed solution of the sample and the analysis reagent is not particularly limited, and can be, for example, set as appropriate depending on the component to be used. The concentration of the same is not particularly limited, and is, for example, in the range of 0.05 to 5 w/v%, preferably in the range of 0.1 to 0.5 w/v%. In the case of using sodium citrate as the reaggregation inhibitor, the concentration of the sodium citrate in the mixed solution of the sample and the analysis reagent is not particularly limited, and is, for example, in the above-mentioned concentration range.

The additive for measuring hemoglobin is not particularly limited, and examples thereof include sodium nitrite, perchloric acid, thiocyanic acid, potassium iodide, potassium bromide, trichloroacetic acid, and trifluoroacetic acid. The concentration of the additive for measuring hemoglobin in the mixed solution of the sample and the analysis reagent is not particularly limited, and is, for example, in the range of 0.01 to 1 w/v%, preferably in the range of 0.01 to 0.1 w/v%. In the case of using the sodium nitrite, the concentration of the sodium nitrite in the mixed solution of the sample and the analysis reagent is not particularly limited, and is, for example, in the above-mentioned concentration range.

In the reaction step of the analysis method of the present invention, a mixed volume ratio (X : Y) of the sample (X) to the analysis reagent (Y) is not particularly limited, and is, for example in the range of 1:0.4 to 1: 99, preferably in the range of 1: 9 to 1: 99, and more preferably in the range of 1 : 19 to 1:49.

In the mixed solution, the amount of the leukocyte-reactive surfactant per 1 $\mu$L of whole blood is, for example, preferably in the range of 0.02 to 2 mg, the amount of the nonionic surfactant per the same is, for example, preferably in the range of 0.001 to 0.25 mg, and the amount of the erythrocyte-reactive surfactant per the same is, for example, preferably in the range of 0.004 to 0.4 mg.

In the reaction step, the time for causing the analysis reagent to react with the leukocyte and erythrocyte by mixing the sample and the analysis reagent is not particularly limited, and is, for example, in the range of 10 to 300 seconds, preferably in the range of 30 to 120 seconds. By setting the reaction time within the above-described time range, the leukocyte reacts with the analysis reagent to such an extent that they can be classified, and the stable reaction state is maintained. Thus, the leukocyte can be analyzed stably.

The form of the analysis reagent of the present invention is not particularly limited, and examples thereof include a liquid, a solid, and a granule. As a method for preparing the analysis reagent, conventionally known producing methods, for example, can be employed, and it is not particularly limited. The preparing method of the liquid analysis reagent is not particularly limited, and the liquid analysis reagent may be prepared by, for example, mixing the above-mentioned surfactant and the above-mentioned buffer. The preparing method of the solid analysis reagent is not particularly limited, and the solid analysis reagent may be prepared by, for example, drying the liquid analysis reagent. The preparing method of the granular analysis reagent is not particularly limited, and the granular analysis reagent may be prepared by, for example, adding an auxiliary reagent such as a granulating agent and the like to the solid analysis reagent and granulating them with a granulator. In the case where the analysis reagent of the present invention is a solid or a granular analysis reagent, components of the analysis reagent may be dissolved, suspended, or dispersed in the mixed solution by mixing the sample and the analysis reagent, or the analysis reagent may be previously dissolved, suspended, or dispersed in a buffer and the like and thereafter mixed with the sample.

Measuring step

In the measuring step, the mixed solution of the sample and the analysis reagent are caused to pass through a fine through-hole, and signals generated at the time of the passage are measured, and leukocytes are classified and counted. It is to be noted that the present invention can be said to be a method for classifying and counting leukocytes by detecting, in the mixed solution of the sample and the analysis reagent, at least one of information about the size of cells in the sample and information about the morphology of cells in the sample. The information can be detected as a signal that will be mentioned later.

The passage of the mixed solution through the fine through-hole and the measurement of the signals generated at the time of the passage are not particularly limited. The fine through-hole may be one part (one point) of a flow path. That is, for example, when the mixed solution is caused to pass through the flow path, using a desired site (one point) of the flow path as the fine through-hole part, signals at the time when the mixed solution passes through this site may be measured. The fine through-hole may be formed by constricting a certain portion excluding both ends of the flow path. That is, "the constricted part" in the flow path may serve as a fine through-hole. In the case where the flow path has the fine through-hole such as mentioned above, the mixed solution of the sample and the analysis reagent is introduced into the flow path from one end thereof to fill the flow path, for example. Further, the sample is caused to move to the other end of the flow path. Thus, the mixed solution is caused to pass through the fine through-hole, and signals generated at the time when the mixed solution passes through the fine through-hole are measured. The method for introducing the mixed solution into the flow path is not particularly limited, and the mixed solution may be introduced by drawing the air with a vacuum pump joined to the other end of the flow path or may be introduced by arranging the fine through-hole between electrodes and applying the voltage between both the electrodes. In the latter case, it is preferable that, for example, the mixed solution is moved by electrophoresis after filling an electrophoretic solution between the electrodes. The flow path is, for example, preferably provided in a cartridge. As the cartridge, a microchip such as a μTAS is preferable.

The fine through-hole may be a through hole (an orifice) provided in a partition separating liquid baths that are joined to each other. In this case, for example, the sample and the analysis reagent are poured into one of the liquid baths, and the other liquid bath is filled with a physiological saline solution. Further, the mixed solution of the sample and the analysis reagent is caused to move to the other liquid bath by depressurizing the liquid bath using a vacuum pump joined to the other liquid bath. Thus, the leukocytes are caused to pass through the fine through-hole, and the signals generated at the time when the mixed solution is passed through the fine through-hole are measured.

The size of the fine through-hole is not particularly limited, and can be set as appropriate depending on the analyzing device to be used. In the case where, for example, the μTAS is used as the analyzing device, a cross-sectional shape of the fine through-hole in the direction perpendicular to the moving direction of the mixed solution is not particularly limited, and examples thereof include rectangular and circular. In the case where the cross-sectional shape is rectangular, the fine through-hole is not particularly limited, and has, for example, a width in the range of 10 to 200 μm and a depth in the range of 10 to 200 μm, preferably a width in the range of 50 to 100 μm and a depth in the range of 50 to 100 μm. In the case where the cross-sectional shape is circular, the pore size (the diameter) of the fine through-hole is, for example, in the range of 10 to 200 μm, preferably in the range of 50 to 100 μm. The length of the fine through-hole in the moving direction is not particularly limited, and is, for example, in the range of 10 to 200 μm, preferably in the range of 50 to 100 μm.

In the case where the fine through-hole is, for example, the through hole (the orifice) provided in the partition, a cross-sectional shape of the through hole in the direction perpendicular to the moving direction of the mixed solution is not particularly limited, and examples thereof include rectangular and circular. In the case where the cross-sectional shape is rectangular, the fine through-hole is not particularly limited, and has, for example, a width in the range of 10 to 200 μm and a height in the range of 10 to 200 μm, preferably a width in the range of 50 to 100 μm and a height in the range of 50 to 100 μm. In the case where the cross-sectional shape is circular, a pore size (diameter) of the fine through-hole is, for example, in the range of 10 to 200 μm, preferably in the range of 50 to 100 μm. The length of the fine through-hole in the moving direction is not particularly limited, and is, for example, in the range of 10 to 200 μm, preferably in the range of 50 to 100 μm.

The signal detected at the time of passing through the fine through-hole is not particularly limited, and examples thereof include an impedance such as a DC impedance or an RF impedance, forward-scattered light, lateral scattered light, and fluorescence.

The method for measuring the signal is not particularly limited, and examples thereof include an electrochemical method for measuring a voltage value or a current value and an optical method for measuring an intensity of light or a dye.

The impedance is, for example, an impedance between electrodes at the time when a solution containing hemocytes passes through the fine through-hole. Specifically, the impedance is an impedance generated at the time when the solution is caused to pass through a fine through-hole arranged between electrodes in the state where a current is applied between both the electrodes. Since the impedance changes at the time when hemocytes such as leukocytes floating in the solution pass through the fine through-hole, leukocytes contained in the sample can be analyzed by measuring an impedance and detecting the change in impedance. The impedance in the case where a direct current is applied as a current is a DC impedance and generally changes depending on the volume of hemocytes. The impedance in the case where a high-frequency current is applied as a current is an RF impedance and generally changes depending on the volume and internal state of hemocytes. In the present invention, the solution containing the hemocytes is a mixed solution containing an analysis reagent of the present invention and a sample. The electrical conductivity of the analysis reagent and the solution containing the hemocytes (i.e., the mixed solution) is not particularly limited, and is, for example, in the range of 11 to 20 mS/cm, preferably in the range of 13 to 18 mS/cm.

The method for detecting the scattering light and the fluorescence is not particularly limited, and for example, general methods can be employed. The detection may be carried out by irradiating hemocytes passing through the fine through-hole with light using a flow cytometer. The light source of the light is not particularly limited, and examples thereof include a water-cooled high-output laser, a low-output air-cooled laser, a diode laser, and a xenon lamp.

The classification and the counting of the leukocytes are not particularly limited, and can be carried out in an appropriate manner using the method capable of analyzing them from information of the signal. In the case where the DC impedance is measured as the signal, leukocytes are classified according to the size from the sizes of detected pulses (changes in impedance), and the proportions of the respective sizes of the leukocytes can be calculated from the frequency of the pulses. The leukocyte can be generally a granulocyte such as a neutrophil, an eosinophil, or a basophil or a mononuclear cell such as a lymphocyte or a monocyte. In the present invention, the classes into which leukocytes are classified are not particularly limited, and are, for example, two classes of granulocytes and mononuclear cells (i.e., two classes of leukocytes), three classes of lymphocytes, monocytes, and granulocytes (i.e., three classes of leukocytes), five classes of neutrophils, eosinophils, basophils, monocytes, and lymphocytes (i.e., five classes of leukosytes). In the analysis method of the present invention, the classification of leukocytes is not particularly limited, and preferably is classification into three classes.

The analysis method of the present invention may be carried out at the same time as the other blood analysis. Objects to be analyzed by the other blood analysis are not particularly limited, and examples thereof include an erythrocyte, hemoglobin, and platelet.

Next, the analysis method of the present invention will be described with reference to embodiments. However, the analysis method of the present invention is not limited to the following embodiments.

(First embodiment)

Hereinafter, one embodiment of the analysis method of the present invention using a cartridge that is a μTAS will be explained. FIGs 1A and 1B show one example of the cartridge in the present embodiment. The cartridge of the present example is a disposable-type cartridge capable of being attached in an analyzing device (not shown). FIG. 1A is a plan view of the cartridge, and FIG. 1B is a perspective view of the same. It is to be noted that in FIGs. 1A and 1B, identical parts are denoted by identical reference numerals. Both FIGs. 1A and 1B are schematic views to provide simplified illustration, and the sizes, the proportions, and the like of each component are not particularly limited to FIGs. 1A and 1B and may be different from those in FIGs. 1A and 1B. As shown in both FIGs. 1A and 1B, the cartridge 1 includes a lower substrate 3, an upper substrate 2, and a connector 7. The connector 7 is arranged on one side surface of a laminate in which the upper substrate 2 is laminated on the lower substrate 3. A wiring pattern (not shown) is formed on the lower substrate 3.

Five through holes are formed in the upper substrate 2. The bottoms of the five through holes are sealed with the lower substrate 3 so that five liquid baths are formed. The five liquid baths are, respectively, a sample introducing part 41, a drain 45, a drain 55, a drain 58, and a drain 65. On the bottom of the upper substrate 2, four large and small concave parts are formed. Two liquid baths are formed by sealing the opening surfaces of the two concave parts among the four concave parts with the lower substrate 3. The two liquid baths are, respectively, a reagent bath 51 and a diluting bath 59. The analysis reagent is sealed in the reagent bath 51. A stirring bar (not shown) is sealed in the diluting bath 59. In the voids formed by sealing the opening surfaces of the remaining two concave parts among the four concave parts with the lower substrate 3, electrodes 61 and 62 connected to an electric wire in the wiring pattern are arranged, respectively. The voids serve as electrode arranging parts. In FIGs. 1A and 1B, the numerals 61 and 62 denote the electrodes and also denote the electrode arranging parts. The plural grooves are formed in the bottom of the upper substrate 2. By sealing opening surfaces of the plural grooves with the lower substrate 3, a flow path is formed so that it links the seven liquid baths composed of the five through holes and the two concave parts and the two electrodes arranging parts so as to communicate with each other.

The sample introducing part 41 communicates with the drain 45 through a sample introducing flow path 42, a branch part 43, and an overflow channel 44 in this order. The sample introducing part 41 communicates also with the diluting bath 59 through a sample metering flow path 46 extending from the branch part 43. An opening of the sample introducing part 41 is an inlet for introducing a sample to be analyzed to the cartridge. An orifice 47 having a small flow path cross-sectional area is formed in the end part of the sample metering flow path 46 on the diluting bath 59 side.

In the cartridge 1, the sample can be metered and introduced into the diluting bath 59 as below, for example. First, the sample is introduced into the sample introducing part 41, and thereafter, the flow path communicating with the drain 45 is depressurized by drawing the air with a vacuum pump and the like (not shown) that is joined to the drain 45. Thus, the sample is introduced from the sample introducing part 41. By the suction, the part of the sample exceeding the volumetric capacity of the sample metering flow path 46 that is between the branch part 43 and the orifice 47 is flown out to the overflow channel 44. Subsequently, the drain 45 is closed, and the flow path communicating with the sample introducing part 41 is pressurized by delivering the air with a vacuum pump and the like (not shown) that is joined to the sample introducing part 41. Thus, the sample whose amount corresponds to the volumetric capacity of the sample metering flow path 46 is introduced into the diluting bath 59. Therefore, the amount of the sample to be introduced is set to the volumetric capacity of the sample metering flow path 46, and the metering and introducing can be achieved.

The reagent bath 51 communicates with the drain 55 through a reagent introducing flow path 52, a branch part 53, and an overflow channel 54 in this order. The reagent bath 51 also communicates with the diluting bath 59 through the reagent metering flow path 56 extending from the branch part 53. An analysis reagent (not shown) is sealed in the reagent bath 51. The reagent metering flow path 56 is branched between the end thereof on the diluting bath side and the diluting bath 59, and the drain 58 is formed on the terminal of the branched part. A diluting part 5 includes the reagent bath 51, the reagent introducing flow path 52, the branch part 53, the overflow channel 54, the drain 55, the reagent metering flow path 56, the drain 58, and the diluting bath 59. It is to be noted that a tapering part 57 is formed on the diluting bath side of the reagent metering flow path 56.

In the diluting part 5, the analysis reagent can be metered and introduced into the diluting bath 59 as below. First, in the state where the drains 55 and 65 are closed, and the drain 58 is opened, the flow path communicating with the reagent bath 51 is pressurized by delivering the air with a vacuum pump and the like (not shown) that is jointed to the reagent bath 51. Thus, the reagent metering flow path 56 is filled with the analysis reagent. Further, the drain 58 is closed, and the drain 65 is opened. A flow path communicating with the drain 55 is pressurized by delivering the air with a vacuum pump (not shown) that is joined to the drain 55. Thus, the analysis reagent whose amount corresponds to the volumetric capacity of the reagent metering flow path 56 is introduced into the diluting bath 59. Thus, the amount of the analysis reagent to be introduced is, for example, set to the volumetric capacity of the reagent metering flow path 56, and metering introduction can be achieved. Furthermore, as mentioned above, the sample is introduced into the diluting bath 59. The sample and the analysis reagent can be mixed by rotating the stirring bar (not shown) in the diluting bath 59 with a magnetic stirrer (not shown). It is to be noted that, in the present example, a surfactant may be added to the analysis reagent so that a hemolytic treatment can be carried out.

A fine through-hole 63 is formed on the flow path that links the diluting bath 59 and the drain 65 so that they communicate with each other. Further, the electrode arranging parts 61 and 62 (two concave parts) are formed respectively in the flow paths that are on the both sides of the fine through-hole 63. The electrode 61 connected to an electric resistance detector (not shown) through the electric wire is arranged in the electrode arranging part 61 (concave part), and the electrode 62 conneced to an electric resistance detector (not shown) through the electric wire is arranged in the electrode arranging part 62 (concave part). A flow rate measuring flow path 64 is formed on the drain 65 side through the fine through-hole 63 and the two electrodes 61 and 62. An analyzing part 6 includes the electrodes 61 and 62, the fine through-hole 63, the flow rate measuring flow path 64, and the drain 65.

In the cartridge 1, the length and the width of the upper substrate 2 are not particularly limited, and are, for example, in the range of 10 to 200 mm, preferably in the range of 20 to 100 mm. The thickness of the upper substrate 2 is not particularly limited, and is, for example, in the range of 0.1 to 10 mm, preferably in the range of 1 to 5 mm.

In the cartridge 1, the length and the width of the lower substrate 3 are not particularly limited, and are, for example, the same as those of the upper substrate 2. The thickness of the lower substrate 3 is not particularly limited, and is, for example, in the range of 0.1 to 10 mm, preferably in the range of 1 to 5 mm.

In the cartridge 1, the materials of the upper substrate 2 and the lower substrate 3 are not particularly limited as long as, for example, the measurement of an absorbance is carried out without any trouble. As the materials of the upper substrate 2 and the lower substrate 3, a material made of a glass, a polymer, or the like can be used, for example. The glass material is not particularly limited, and examples thereof include synthetic quartz glasses, molten silica, and borosilicate glasses. The polymer material is not particularly limited, and examples thereof include acrylic resins such as polymethyl methacrylate (PMMA), cycloolefin polymers (COPs), polycarbonates (PCs), polydimethylcyclohexanes (PDMSs), polystyrenes (PSs), polylactic acids (PLAs), epoxy resins, polyethylenes (PEs), polytetrafluoroethylenes (PTFEs), and polyetheretherketones (PEEKs). In the lower substrate 3, plural substrates made of the above-described materials are laminated. Wiring patterns formed of copper foils are formed between the plural substrates.

In the cartridge 1, the diameter and the depth of the sample introducing part 41 are not particularly limited. For example, the diameter is in the range of 0.1 to 10 mm and the depth is in the range of 0.1 to 10 mm. Preferably, the diameter is in the range of 1 to 5 mm and the depth is in the range of 1 to 5 mm.

In the cartridge 1, the diameter and the depth of the reagent bath 51 are not particularly limited. For example, the diameter is in the range of 0.5 to 50 mm and the depth is in the range of 0.1 to 10 mm. Preferably, the diameter is in the range of 1 to 20 mm and the depth is in the range of 1 to 5 mm.

In the cartridge 1, the diameter and the depth of the diluting bath 59 are not particularly limited. For example, the diameter is in the range of 0.5 to 50 mm and the depth is in the range of 0.1 to 10 mm. Preferably, the diameter is in the range of 1 to 10 mm and the depth is in the range of 1 to 5 mm.

In the cartridge 1, the diameter and the depth of each of the drains 45, 55, 58, and 65 are not particularly limited. For example, the diameter is in the range of 0.1 to 10 mm and the depth is in the range of 0.1 to 10 mm. Preferably, the diameter is in the range of 1 to 5 mm and the depth is in the range of 1 to 5 mm.

In the cartridge 1, the shapes of the sample introducing part 41, the reagent bath 51, the diluting bath 59, and the liquid baths of the drains 45, 55, 58, and 65 are cylindrical. However, the present invention is not limited to this. In the present invention, the shapes of the respective liquid baths are not particularly limited, and examples thereof include cylindrical, quadrilateral prism, quadrilateral pyramid, and conical. The shapes of the respective liquid baths may be identical to or different from each other and are not particularly limited.

In the cartridge 1, the width and the depth of the reagent metering flow path 56 are not particularly limited. For example, in the part having the maximum cross-sectional area, the width is in the range of 0.1 to 10 mm and the depth is in the range of 0.1 to 10 mm. Preferably, in the same, the width is in the range of 0.5 to 5 mm and the depth is in the range of 0.1 to 5 mm.

In the cartridge 1, the width and the depth of the orifice 47 are not particularly limited. For example, the width is in the range of 1 to 200 $\mu$m and the depth is in the range of 0.1 to 10 mm. Preferably, the width is in the range of 10 to 100 $\mu$m and the depth is in the range of 0.1 to 5 mm.

In the cartridge 1, the width, the depth, and the length of the fine through-hole 63 are the same as mentioned above.

In the cartridge 1, the width and the depth of flow path other than the reagent metering flow path 56, the orifice 47, and the fine through-hole 63 are not particularly limited. For example, the width is in the range of 10 to 1000 $\mu$m and the depth is in the range of 0.1 to 10 mm. Preferably, the width is in the range of 100 to 500 $\mu$m and the depth is in the range of 0.1 to 5 mm.

In the cartridge 1, the maximum thickness of the whole cartridge refers to the sum of the thicknesses of the upper substrate 2 and the lower substrate 3. The thicknesses of the upper substrate 2 and the lower substrate 3 are the same as mentioned above.

The method for producing the cartridge 1 is not particularly limited, and for example, the conventionally known methods can be employed.

Next, the leukocyte analysis method of the present example using the cartridge 1 will be described.

First, the cartridge 1 is attached in the analyzing device (not shown) through a connector 7. Human whole blood as a sample is introduced from the sample introducing part 41 as mentioned above. Further, as mentioned above, the amount of the human whole blood corresponding to the volumetric capacity of the sample metering flow path 46 is metered and introduced into the diluting bath 59. Furthermore, as mentioned above, the analysis reagent whose amount corresponds to the volumetric capacity of the reagent metering flow path 56 is metered and introduced into the diluting bath 59. The introduced sample and analysis reagent are mixed in the diluting bath 59, and are stirred by rotating the stirring bar (not shown) with the magnetic stirrer (not shown), thereby causing the leukocytes and erythrocytes in the sample and the analysis reagent to react with each other.

Next, a voltage is applied between the electrodes 61 and 62, and the air is drawn with a vacuum pump and the like (not shown) that is joined to the drain 65 to depressurize the inside of the flow path communicating with the drain 65. Thus, a solution (a mixed solution) in which the sample and the analysis reagent are mixed is caused to pass through the fine through-hole 63 and further is caused to flow out to the flow rate measuring flow path 64. The pulses (changes in impedance) generated at the time when the mixed solution passes through the fine through-hole 63 are measured using an electric resistance detector (not shown), and a histogram in which the size and the pulse frequency are represented on the respective two axes is prepared. The leukocytes are classified into three classes according to the size thereof from an inflected state of the prepared histogram, and the respective pulse frequencies of the classes are added up. The pulse frequency obtained by adding up is divided by the flow rate at which the mixed solution has flown out to the flow rate measuring flow path 64, and thus, the proportions of the respective leukocytes classified into three classes in the sample are calculated. It is to be noted that the three classes of the leukocytes are, in ascending order of the pulse, lymphocytes, monocytes, and granulocytes.

(Second embodiment)

Next, another embodiment of the analysis method of the present invention will be explained. First, one example of a leukocyte analysis device used in the analysis method of this embodiment is shown in FIG. 2. FIG. 2 is a cross-sectional view of the leukocyte analysis device. FIG. 2 is a schematic view to provide simplified illustration, and the size, the proportion, and the like of each component are not particularly limited to FIG. 2 and may be different from those in FIG. 2.

As shown in FIG. 2, the leukocyte analysis device 8 includes two baths of a main bath 9 and a sub bath 10, a sucking part 11, two electrodes of electrodes 12 and 13, and an analyzing part (not shown). The main bath 9 and the sub bath 10 are arranged so as to be separated with a partition. A through hole serving as a fine through-hole 14 is formed in the partition, and links the two baths so that the insides thereof communicate with each other. The upper surface of the main bath 9 is open. In the sub bath 10, a through hole is formed in a side surface that is different from the partition, and the sub bath 10 communicates with the sucking part 11 through the through hole. A pump (not shown) is arranged on the other side of the sucking part 11. The electrode 12 is arranged in the main bath 9, and the electrode 13 is arranged in the sub bath 10. The electrodes 12 and 13 are arranged in the terminal of an electric wire (partially shown in FIG. 2) joined to the analyzing part (not shown).

As the materials of the main bath 9 and the sub bath 10, the same material as that of the upper substrate of the above-mentioned cartridge and the like can be used. The shapes of the main bath 9 and the sub bath 10 are not particularly limited, and examples thereof include rectangular and cylindrical. The shapes of the main bath 9 and the sub bath 10 may be identical to or different from each other and are not particularly limited. The upper surface of the main bath 9 may be open as mentioned above.

The fine through-hole 14 is, for example, a through hole having a diameter in the range of 1 to 500 $\mu$m and a length in the range of 1 to 200 $\mu$m, preferably a diameter in the range of 10 to 100 $\mu$m and a length in the range of 10 to 100 $\mu$m. It is to be noted that the diameter is, for example, a diameter of the cross section that is perpendicular to the direction in which the fine through-hole 14 passes through, and the length is a length in the direction in which the fine through-hole 14 passes through and corresponds to a thickness of the partition separating the main bath 9 and the sub bath 10.

The material of the sucking part 11 is not particularly limited, and the same materials as those of the above-mentioned two baths can be used. The shape of the sucking part 11 is not particularly limited, and is, for example, cylindrical.

The method for producing the leukocyte analysis device 8 of the present example is not particularly limited, and conventionally know methods may be used as appropriate, for example.

Next, the leukocyte analysis method of the present example using the leukocyte analysis device 8 will be described.

First, the analysis reagent of the present invention is poured into the main bath 9 and the sub bath 10, and thereafter, human whole blood as a sample is added to the main bath 9, and they are mixed together for 30 seconds. Next, a voltage is applied between the two electrodes, and the sub bath 10 is depressurized by drawing the air with the pump that is joined to the sucking part 11. Thus, the mixed solution is caused to pass through the fine through-hole 14 and to move from the main bath 9 to the sub bath 10. The pulses (changes in impedance) generated at the time when the mixed solution passes through the fine through-hole 14 are measured using the analyzing part (not shown). In the same manner as in the first embodiment, a histogram is prepared, leukocytes are classified and counted, and the proportions of the classified leukocytes are calculated.

(Third embodiment)

Next, yet another embodiment of the analysis method of the present invention will be described. One example of the leukocyte analysis device used in the analysis method of this embodiment is shown in FIG. 7. FIG. 7 is a cross-sectional view of the leukocyte analysis device. FIG. 7 is a schematic view to provide a simplified illustration, and the size, the proportion, and the like of each component are not particularly limited to FIG. 7 and may be different from those in FIG. 7.

As shown in FIG. 7, the leukocyte analysis device 88 includes a main bath part 90, a sub bath part 100, a stirring part 160, a sucking part 11, and electrodes 12 and 13. The stirring part 160 includes a substrate part 16 and a stirring pipe 18. The sucking part 11 includes a sucking pipe 19 and a syringe (not shown).

The main bath part 90 includes a two-layer laminate in which two substrates of a substrate 91a and a substrate 91b are laminated so that the two substrates are arranged in parallel, and it includes a void part 9 whose upper side is open and capable of contain a liquid. This void part 9 serves as a main bath 9. In the main bath part 90, a through hole 22c communicating with the inside of the main bath 9 is formed in the substrate 91a that is a side surface of the main bath 9, and a through hole 22a communicating with the inside of the main bath 9 is formed in the substrate 91b that is a side surface of the main bath 9. One end of the stirring pipe 18 is joined to the through hole 22c. The electrode 12 is arranged in the main bath 9. The electrode 12 is joined to a voltage supply through an electric wire (not shown).

The sub bath part 100 is composed of a two-layer laminate in which two substrates of a substrate 101a and a substrate 101b are laminated so that the two substrates are arranged in parallel and is arranged adjacent to the main bath part 90. The sub bath part 100 includes a void part 10 into which liquid can be introduced. This void part serves as a sub bath 10. In the sub bath part 100, three through holes communicating with the inside of the sub bath 10 are formed along a longitudinal direction in a substrate 101a that is a side surface of the sub bath 10. Among the three through holes, the middle through hole 22b is joined to a through hole 22a of the main bath part 90, adjacent to the through hole 22b. Thus, the inside of the main bath part 90 and the inside of the sub bath part 100 communicate with each other. It is to be noted that in the leukocyte analysis device 88 of the present example, the joining part between the through hole 22b of the sub bath part 100 and the through hole 22a of the main bath part 90 serves as a fine through-hole 14. Among the three through holes, the upper through hole 21a serves as an inlet 21a, and the lower through hole 21b serves as an inlet 21b. One end of a sucking pipe (not shown) is joined to the inlet 21a, and the syringe (not shown) is joined to the other end of the sucking pipe. The inlets 21a and 21b may be openable and closable or may be joined to a solution bath for discharging a solution that is introduced into the sub bath part 100. In the void part 10, a void portion that allows the inlet 21a and the through hole 22b to communicate with each other can also be, for example, used as a quantitative determination part 15. The electrode 13 is arranged in the sub bath 10. The electrode 13 is joined to the voltage supply through an electric wire (not shown).

FIG. 8 shows another example of the fine through-hole 14 part of the leukocyte analysis device 88. FIG. 8 is an enlarged view showing another example of the part within the dotted line shown in FIG. 7. As shown in FIG. 8, an elastic body 840 having a through hole may be arranged in a region in which the through hole 22a of the main bath part 90 and the through hole 22b of the sub bath part 100 are joined to each other. In this case, the through hole formed in the elastic body 840 serves as the fine through-hole 84. The material of the elastic body 840 is not particularly limited, and can be, for example, a silicon rubber or the like.

The substrate part 16 is composed of a two-layer laminate in which two substrates of a substrate 161a and a substrate 161b are laminated so that the two substrates are arranged in parallel, and it includes a void part 17 capable of containing liquid in the inside thereof. This void part serves as a stirring bath 17. In the substrate part 16, two through holes of through holes 22d and 22e that communicate with the inside of the stirring bath 17 are formed along a longitudinal direction in the substrate 161b that is a side surface of the stirring bath 17. One end of the sucking pipe 19 is joined to the through hole 22d, and the syringe (not shown) is joined to the other end of the sucking pipe 19. One end of the stirring pipe 18 is joined to the through hole 22e, and the other end of the same is, as mentioned above, joined to the through hole 22c of the main bath part 90. The inside of the substrate part 16 and the inside of the main bath part 90 communicate with each other through this stirring pipe 18.

In the leukocyte analysis device 88 of the present example, the materials of the substrates 91a, 91b, 101a, 101b, 161a, and 161b are not particularly limited, and examples thereof include the above-mentioned glasses and polymers.

The size of the main bath part 90 is not particularly limited, and is, for example, in the range of 3 to 100 mm in height, in the range of 3 to 50 mm in width, and in the range of 3 to 50 mm in thickness. The volume of the main bath 9 is not particularly limited, and is, for example, in the range of 10 to 5000 μL, preferably in the range of 100 to 1000 μL.

The size of the sub bath part 100 is not particularly limited, and is, for example, in the range of 10 to 200 mm in height, in the range of 3 to 50 mm in width, and in the range of 3 to 50 mm in thickness. The volume of the sub bath 10 is not particularly limited, and is, for example, in the range of 10 to 5000 μL, preferably in the range of 100 to 1000 μL. A void part between the through hole 22b and the inlet 21a can be a quantitative determination part 15. In this case, the volume of the quantitative determination part 15 is preferably set to a desired volume that is suitable for quantitative determination.

The size of the substrate part 16 is not particularly limited, and is, for example, in the range of 3 to 200 mm in height, in the range of 3 to 50 mm in width, and in the range of 3 to 50 mm in thickness. The volume of the stirring bath 17 is not particularly limited, and is, for example, preferably in the range of 100 to 1000 μL.

The size of the fine through-hole 14 is, for example, in the range of 1 to 500 μm in diameter and in the range of 1 to 200 μm in length, preferably in the range of 10 to 200 μm in diameter and in the range of 10 to 100 μm in length. It is to be noted that the diameter is a diameter of a cross section that is perpendicular to the direction in which the fine through-hole 14 passes through, and the length is the sum of the thickness of the substrate 91a and the thickness of the substrate 101a in the fine through-hole 14 part. Also in FIG. 8, the size of the fine through-hole 84 is, for example, the same as above.

The diameter of each of the inlets 21a and 21b is, for example, in the range of 0.1 to 2 mm, preferably in the range of 0.5 to 1 mm. The diameter of each of the through holes 22c, 22d, and 22e is, for example, in the range of 0.1 to 2 mm, preferably in the range of 0.5 to 1 mm.

The materials of the stirring pipe 18 and the sucking pipe 19 are not particularly limited, and for example, a stainless steel or the like can be used. The stainless steel is not particularly limited, and can be, for example, SUS or the like. The shapes of the stirring pipe 18 and the sucking pipe 19 are not particularly limited, and are, for example, cylindrical. In the case where the shapes are cylindrical, the inside diameter of each of the stirring pipe and the sucking pipe is, for example, in the range of 1 to 500 μm, preferably in the range of 10 to 200 μm. The length of each of the stirring pipe and the sucking pipe is not particularly limited, and is, for example, in the range of 1 to 500 mm, preferably in the range of 10 to 200 mm.

The method for producing the leukocyte analysis device 88 of the present example is not particularly limited, and for example, conventionally known methods may be used as appropriate.

Next, the leukocyte analysis method of the present example using the leukocyte analysis device 88 will be described.

First, the sample is prepared by diluting human whole blood with a physiological saline solution. The physiological saline solution is introduced from the inlet 21b using a syringe (not shown) until it reaches the upper part of the electrode 13 that is in the sub bath 10, and the inlet 21b is closed. The sample is poured into the main bath 9. The baths and pipes communicating with the main bath 9 are depressurized (the air is sucked) by a syringe (not shown) that is joined to the sucking pipe 19. Thus, the sample in the main bath 9 is introduced into the stirring bath 17. Next, the analysis reagent of the present invention is poured into the main bath 9. The baths and pipes communicating with the main bath 17 are pressurized (the air is deliverd) by the syringe. Thus, the sample in the stirring bath 17 is reintroduced into the main bath 9. Further, depressurizing and pressurizing the baths and pipes communicating with the main bath 9 are alternatively repeated by the syringe. Thus, the sample and the analysis reagent are mixed. After the mixing, the mixed solution of the sample and the analysis reagent is allowed to stand still in the main bath 9. The voltage is applied between the electrodes 12 and 13, and the sub bath 10 and the main bath 9 are depressurized by suction with a syringe (not shown) that is joined to the inlet 21a. Thus, the mixed solution is caused to pass through the fine through-hole 14 and to move from the main bath 9 to the sub bath 10. Pulses (changes in impedance) at the time when the sample passes through the fine through-hole 14 are measured using an analyzing part (not shown). In the same manner as in the first embodiment, a histogram is prepared, leukocytes are classified and counted, and the proportions of the classified leukocytes are calculated. In the void part 10 of the sub bath part 100, in the case where a void part between the through hole 22b and the inlet 21a is used as a quantitative determination part 15, the mixed solution that has passed through the main bath part 90 can be quantitatively determined. Examples

Next, examples of the present invention will be described together with comparative examples. However, the present invention is not limited to the following examples.

(Example 1)

In Example 1, leukocytes were measured by the following procedure using human whole blood with a hematocrit value of 31.4% as a sample. The above-mentioned leukocyte analysis device 8 shown in FIG. 2 was used for the measurement of leukocytes in the present example. In the leukocyte analysis device 8, the fine through-hole 14 is a through hole with a diameter of 50 $\mu$m and a length of 60 $\mu$m.

First, an analysis reagent having a composition shown in Table 1 below was prepared. An electrical conductivity of the analysis reagent was adjusted using sodium chloride so as to be 11.33 mS/cm.

(Table 1) Analysis reagent

| Component | Compounding concentration |
| --- | --- |
| ADA-buffer (pH 7.4) | 30mmol/L |
| lauryltrimethylammonium chloride | 0.75 w/v% |
| cetyltrimethylammonium chloride | 0.1 w/v% |
| Saponin | 0.18 w/v% |
| sodium citrate | 0.25 w/v% |
| sodium nitrite | 0.05 w/v% |
| sucrose monolaurate | 0.0125 w/v% |

Next, 2400 $\mu$L of the analysis reagent was poured into the main bath 9, and the sub bath 10 was filled with the analysis reagent. 100 $\mu$L of the whole blood was added to the main bath 9 and stirred so that the whole blood was caused to react with the analysis reagent. In Example 1, the dilution ratio of the whole blood was 25. That is, in Example 1, leukocytes were analyzed under the condition where a sample dilution ratio was low. Next, after allowing the sample and the analysis reagent to react for 30 seconds, a voltage of 5 V was applied between the electrode 12 arranged in the main bath 9 and the electrode 13 arranged in the sub bath 10, and the air in the main bath 9 and the sub bath 10 was drawn at a flow velocity of 14$\mu$L/30 sec for 10 seconds by a pump in the sucking part 11. Pulses generated at the time when a mixed solution of the sample and the analysis reagent passed through the fine through-hole 14 during the 10 seconds were measured by a self-produced electronic detector (not shown). A histogram showing a particle size distribution of leukocytes was prepared, in which the horizontal axis represents the obtained pulse height and the vertical axis represents the obtained pulse number. The leukocytes were classified into three sizes: a small size (lymphocytes), a middle size (monocytes), and a large size (granulocytes), according to their pulse heights. In the classification, leukocytes falling within a mountain-shape part appearing next to a mountain-shape part denoting an electrical noise at the left end of the histogram were classified as the small size. Leukocytes falling within a mountain-shape part extending from a second mountain-shape part counted from the mountain-shape part denoting the electrical noise to the right end of the histogram were classified as the large size. Leukocyte falling within a valley-shape part appearing between the mountain-shape part denoting the small size and the mountain-shape part denoting the large size were classified as the middle size. The pulse numbers of the respective three sizes were added up, and the respective three proportions (n) in the total pulse number were calculated.

Further, the proportions (m) of the leukocytes of the respective sizes were calculated in the same manner as mentioned above except that the pulses obtained for 10 seconds after a lapse of 60 seconds from the start of the reaction were measured. Then, with respect to the leukocytes of each size, the proportion change rate k (%) between the proportion obtained when the reaction time was 30 seconds and the proportion obtained when the reaction time was 60 seconds was calculated using the following equation (1).

$$\mathrm{k}\ (\%) = \mathrm{m/n} \times 100 \qquad \cdots (1)$$

m = proportion (%) of leukocytes of each size in the case where the reaction time was 60 seconds.

n = proportion (%) of leukocytes of each size in the case where the reaction time was 30 seconds.

(Example 2)

In Example 2, leukocytes were measured, and the proportions and the change rates were calculated in the same manner as in Example 1 except that human whole blood with a hematocrit value of 39.2% was used as the sample.

The respective proportions and change rates of the leukocytes of the respective sizes in Examples 1 and 2 are shown in Table 2 below. Histograms showing particle size distributions of leukocytes of Example 1 are shown in FIGs. 3A to 3C. FIG. 3A is a histogram showing a particle size distribution in the case where the reaction time was 30 seconds. FIG. 3B is a histogram showing a particle size distribution in the case where the reaction time was 60 seconds. FIG. 3C shows the state where these two histograms were superimposed. Histograms showing particle size distributions of leukocytes of Example 2 are shown in FIGs. 4A to 4C. As in Example 1, FIG. 4A is a histogram showing a particle size distribution of leukocytes in the case where the reaction time was 30 seconds. FIG. 4B is a histogram showing a particle size distribution of leukocytes in the case where the reaction time was 60 seconds. FIG. 4C shows the state where these two histograms were superimposed. In FIGs. 3C and 4C, a solid line (a) denotes a histogram in the case where the reaction time was 30 seconds, and a solid line (b) denotes a histogram in the case where the reaction time was 60 seconds. In the histograms of FIGs. 3A to 3C and 4A to 4C, the horizontal axis represents the pulse height, while the vertical axis represents the pulse number. In the histograms of FIGs. 3A, 3B, 4A, and 4B, leukocytes fall within a pulse height range of a-b were classified as the small size (lymphocytes), leukocytes fall within a pulse height range of b-c were classified as the middle size (monocytes), and leukocytes fall within a pulse height range of c or more (right side of c) were classified as the large size (granulocytes).

[Table 2]

| | Example 1 | | | Example 2 | | |
|---|---|---|---|---|---|---|
| | Proportion (%) | | Change rate (%) | Proportion (%) | | Change rate (%) |
| | Reaction 30 sec | Reaction 60 sec | | Reaction 30 sec | Reaction 60 sec | |
| Small size | 30.1 | 31.1 | 103.4 | 33.8 | 34.0 | 100.7 |
| Medium size | 8.1 | 9.2 | 113.2 | 6.3 | 6.8 | 107.4 |
| Large size | 61.8 | 59.7 | 96.6 | 59.9 | 59.2 | 98.8 |
| Average change rate | - | - | 104.4 | - | - | 102.3 |

As shown in FIGs. 3C and 4C, in both Examples 1 and 2, the difference between the respective histograms that are different in reaction time from each other was small, and as shown in Table 2 above, in both Examples 1 and 2, the change rates denoting the difference between the respective proportions in the respective reaction times were approximately 100%. As above, in the case where the analysis reagent containing sucrose monolaurate was used, the difference between the respective measurement results in the respective reaction times of the sample and the analysis reagent was small so that leukocytes could be measured stably. In Examples 1 and 2, whole bloods with the different hematocrit values from each other were used. However, the results as shown above were obtained in both Examples 1 and 2. Thus, it is found out that leukocytes can be measured stably while avoiding the influence of a hematocrit value.

(Comparative Example 1)

In Comparative Example 1, leukocytes were measured, and the proportions and the change rates were calculated in the same manner as in Example 1 except that an analysis reagent used corresponded to the analysis reagent used in Example 1 from which only sucrose monolaurate was eliminated.

(Comparative Example 2)

In Comparative Example 2, leukocytes were measured, and the proportions and the change rates were calculated in the same manner as in Comparative Example 1 except that human whole blood with a hematocrit value of 39.2% was used as the sample.

The proportions and the change rates in Comparative Examples 1 and 2 are shown in Table 3 below. Histograms showing particle size distributions of leukocytes of Comparative Example 1 are shown in FIGs. 5A to 5C. FIG. 5A is a histogram showing a particle size distribution in the case where the reaction time was 30 seconds. FIG. 5B is a histogram showing a particle size distribution in the case where the reaction time was 60 seconds. FIG. 5C shows the state where these two histograms were superimposed. Histograms showing particle size distributions of leukocytes of Comparative Example 2 are shown in FIGs. 6A to 6C. As in Example 1, FIG. 6A is a histogram showing a particle size distribution of leukocytes in the case where the reaction time was 30 seconds. FIG. 6B is a histogram showing a particle size distribution of leukocytes in the case where the reaction time was 60 seconds. FIG. 6C shows the state where these two histograms were superimposed. In FIGs. 5C and 6C, a solid line (a) denotes a histogram in the case where the reaction time was 30 seconds, and a solid line (b) denotes a histogram in the case where the reaction time was 60 seconds. In the histograms of FIGs. 5A to 5C and 6A to 6C, the horizontal axis represents the pulse height, while the vertical axis represents the pulse number. In the histograms of FIGs. 5A, 5B, 6A, and 6B, leukocytes fall within a pulse height range of a-b were classified as the small size (lymphocytes), leukocytes fall within a pulse height range of b-c were classified as the middle size (monocytes), and leukocytes fall within a pulse height range of c or more (right side of c) were classified as the large size (granulocytes).

[Table 3]

| | Comparative Example 1 | | | Comparative Example 2 | | |
|---|---|---|---|---|---|---|
| | Proportion (%) | | Change rate (%) | Proportion (%) | | Change rate (%) |
| | Reaction 30 sec | Reaction 60 sec | | Reaction 30 sec | Reaction 60 sec | |
| Small size | 28.8 | 19.9 | 69.2 | 33.1 | 33.7 | 102.0 |
| Medium size | 1.9 | 3.8 | 197.8 | 7.9 | 7.0 | 89.0 |
| Large size | 69.3 | 76.3 | 110.1 | 59.1 | 59.3 | 100.3 |
| Average change rate | - | - | 125.7 | - | - | 97.1 |

In Comparative Example 1, as shown in FIG. 5C, the difference between the respective histograms that are different in reaction time from each other was large, and as shown in Table 3 above, the change rates were 69.2% to 197.8% and thus exhibited variations, and the leukocytes could not be analyzed stably. On the other hand, in Comparative Example 2, the difference between the respective measurement results in the respective reaction times of the sample and the analysis reagent was small as compared to Comparative Example 1, and as shown in Table 3, the change rates were 89.0% to 102.0% and thus exhibited a small range of variations, and the leukocytes could be analyzed stably. In Comparative Examples 1 and 2 using the analysis reagent excluding sucrose monolaurate, when the respective hematocrit values of the samples were different from each other, the variations in measurement results were generated, so that the leukocytes could not be analyzed stably.

It was found from the above result that even when dilution ratio was low, and the flow rate was slow, the leukocytes could be analyzed stably without the influence of a hematocrit value by the use of the analysis reagent containing sucrose monolaurate as in Examples 1 and 2. On the other hand, when the analysis reagent not containing sucrose monolaurate was used as in Comparative Examples 1 and 2 in the case where the dilution ratio was low, and the flow velocity was slow, the measurement was influenced by the hematocrit value, the variations in the measurement results were generated, and the leukocytes could not be analyzed stably.

(Example 3)

In Example 3, leukocytes were measured by the following procedure using an analysis reagent to which each additive reagent shown in Table 4 below was added. It is to be noted that the leukocyte analysis device 88 shown in FIG. 7 was used in the measurement in Example 3. In the leukocyte analysis device 88, a region including a fine through-hole is as shown in FIG. 8. In the leukocyte analysis device 88, a volumetric capacity of the main bath 9 was about 700 $\mu$L, a volumetric capacity of the sub bath 10 was about 100 $\mu$L, a volumetric capacity of the quantitative determination part 15 was about 300 $\mu$L, and a volumetric capacity of the stirring bath 17 was about 400 $\mu$L. The fine through-hole 84 shown in FIG. 8 was a through hole with a diameter of 100 $\mu$m and a length of 100 $\mu$m. As the electrodes 12 and 13, electrodes each having a diameter of 1$\mu$m and made of platinum were used. A voltage of 30 V was applied between the electrodes 12 and 13 using a voltage supply. Pulses (changes in impedance) generated at the time when the sample passes through the fine through-hole 84 were measured using an amplifier set at 30 V and 5 gain. Human whole blood with a hematocrit value of 41.6% was used as a blood sample. In Example 3, the human whole blood was diluted with a physiological saline solution (0.9 w/v%, produced by Nacalai Tesque, Inc.) by a factor of 100. Thus, a diluted sample was prepared.

(Table 4)

| Example | Additive reagent | Manufacturer name |
|---|---|---|
| Example 3-1 | sucrose laurate | Dojindo laboratories |
| Example 3-2 | sucrose monolaurate | Dojindo laboratories |
| Example 3-3 | sucrose monocaprate | Dojindo laboratories |
| Example 3-4 | dodecyl maltoside | Dojindo laboratories |
| Comparative Example 3-1 | additive reagent-free | |
| Comparative Example 3-2 | lauryltrimethylammonium chloride | Nacalai Tesque, Inc. |
| Comparative Example 3-3 | polyoxyethylene lauryl ether (30% solution) (product name: Brig 35) | Nacalai Tesque, Inc. |

First, a basic reagent having a composition shown in Table 5 below was prepared. In Table 5, the compounding concentration is a concentration of each component in an analysis reagent. Further, in Table 5, the final concentration is a concentration in a mixed solution obtained by mixing the analysis reagent of the present example and the sample. An electrical conductivity of the basic reagent was adjusted using sodium chloride so as to be 18 mS/cm. The respective analysis reagents of Examples 3-1 to 3-4 and Comparative Examples 3-1 to 3-3 were prepared by adding the respective additive reagents listed in Table 4 so that its concentration becomes 0.1 w/v%. In Example 3, the sucrose laurate added to the analysis reagent of Example 3-1 was a mixture containing about 80% to 90% by volume of sucrose monolaurate (monoester) and about 10% to 20% by volume of sucrose dilaurate (diester) or sucrose trilaurate (triester).

(Table 5) Basic reagent

| Component | Compounding concentration (mmol/L) | Final concentration (mmol/L) |
|---|---|---|
| phosphate buffer (pH 7.5) | 83 | 25 |
| lauryltrimethylammonium chloride | 43 | 12.88 |
| cetyltrimethylammonium chloride | 2 | 0.625 |

Next, 350 $\mu$L of the diluted sample was poured into the main bath 9. The sample was introduced into the stirring bath 17 by depressurization (suction of the air) at the suction rate of 400 $\mu$L/sec by the syringe (not shown) joined to the sucking pipe 19 of the leukocyte analysis device 88. Next, 150 $\mu$L of the analysis reagent was poured into the main bath 9. The sample was introduced into the main path 9 by pressurization (delivery of the air) at the suction rate of 400 μL/sec by the syringe. By the syringe, an operation of drawing 350 μL of the air and then delivering 350 μL of the air at the rate of 400 μL/sec was repeated two more times. Thus, the sample and the analysis reagent were mixed. This mixed solution was allowed to stand still in the main bath 9 for 1 minute. The concentration (final concentration) of each additive reagent in the mixed solution was 0.03 w/v%. Further, a voltage was applied between the electrodes 12 and 13, and 300 μL of the mixed solution was moved from the main bath 9 to the sub bath 10 at the rate of about 2.8 μL/sec by delivering the air by the syringe. The pulses (changes in impedance) generated at the time when the sample passes through the fine through-hole 84 were measured by a self-produced electronic detector (not shown). Seven pulse data were subjected to a moving average calculation, and a histogram showing a particle size distribution of leukocytes was prepared, in which the horizontal axis represents the pulse height that was subjected to the moving average calculation and the vertical axis represents the pulse number that was subjected to the same. The leukocytes were classified into three sizes: a small size (lymphocytes), a middle size (monocytes), and a large size (granulocytes) according to their pulse heights. In the classification, leukocytes falling within a mountain-shape part appearing next to a mountain-shape part denoting an electrical noise at the left end of the histogram were classified as the small size. Leukocytes falling within a mountain-shape part extending from a second mountain-shape part counted from the mountain-shape part denoting the electrical noise to the right end of the histogram were classified as the large size. Leukocytes falling within a valley-shape part appearing between the mountain-shape part denoting the small size and the mountain-shape part denoting the large size were classified as the middle size. The pulse numbers of the respective three sizes were added up, and the respective three proportions (n) in the total pulse number were calculated.

In FIGs. 9 and 10 and Table 6, the analysis results of the leukocytes using the respective analysis reagents to which the respective additive reagents described in Table 4 were added are shown. FIGs. 9A to 9C are histograms showing particle size distributions of leukocytes using the analysis reagents of the respective comparative examples. FIG. 9A is a histogram showing a result of analysis using the analysis reagent of Comparative Example 3-1 (basic reagent). FIG. 9B is a histogram showing a result of analysis using the analysis reagent of Comparative Example 3-2 (the analysis reagent to which lauryl trimethyl ammonium chloride was added). FIG. 9C is a histogram showing a result of analysis using the analysis reagent of Comparative Example 3-3 (the analysis reagent to which polyoxyethylene lauryl ether was added). FIGs. 10A to 10D are histograms showing particle size distributions of leukocytes using the analysis reagents of the respective examples. FIG. 10A is a histogram showing a result of analysis using the analysis reagent of Example 3-1 (the analysis reagent to which sucrose laurate was added). FIG. 10B is a histogram showing a result of analysis using the analysis reagent of Example 3-2 (the analysis reagent to which sucrose monolaurate was added). FIG. 10C is a histogram showing a result of analysis using the analysis reagent of Example 3-3 (the analysis reagent to which sucrose monocaprate was added). FIG. 10D is a histogram showing a result of analysis using the analysis reagent of Example 3-4 (the analysis reagent to which dodecyl maltoside was added). In histograms shown in FIGs. 9A to 9C and 10A to 10D, the horizontal axis represents the pulse height, while the vertical axis represents the pulse number. In the histograms, the leukocytes with the pulse heights in area 2 were classified as the small size (lymphocytes), the leukocytes with the pulse heights in area 3 were classified as the middle size (monocytes), and the leukocytes with the pulse heights in area 4 were classified as the large size (granulocytes). It is to be noted that the area 1 denotes a blood component (noise) other than the leukocytes, and a main component thereof can be, for example, lysate of erythrocytes. The following Table 6 shows, with respect to the respective areas, pulse ranges, pulse numbers, and their proportions obtained by the respective analyses using the respective analysis reagents.

As shown in FIG. 9A, in the case where the basic reagent of Comparative Example 3-1 to which a nonionic surfactant was not added was used, the valley between the area 1 (noise) and the area 2 (lymphocytes) became shallow, and the separation accuracy of the area 1 (noise) and the area 2 (lymphocytes) became low. Further, as shown in FIG. 9B, in the case where the analysis reagent of Comparative Example 3-2 to which lauryl trimethyl ammonium chloride was added was used, the valley between the area 1 (noise) and the area 2 (lymphocytes) became shallow, and the peak in the area 4 (granulocyte) became lower than that in the case where the basic reagent was used. Furthermore, as shown in FIG. 9C, in the case where the analysis reagent of Comparative Example 3-3 to which polyoxyethylene lauryl ether was added was used, the valley between the area 1 (noise) and the area 2 (lymphocytes) became shallow, and the peak in the area 2 (lymphocytes) became low. On the other hand, as shown in FIGs. 10A to 10D, in the case where the analysis reagents of Examples 3-1 to 3-4 to which the nonionic surfactants, namely, sucrose laurate, sucrose monolaurate, sucrose monocaprate, and dodecyl maltoside, were added, respectively, the valley between the area 1 (noise) and the area 2 (lymphocytes) became deep, the peak in the area 2 (lymphocytes) did not become low, and the peak in area 4 (granulocytes) became more clear, as compared with those in the case where the basic reagent was used. That is, by adding the nonionic surfactant, the separation accuracy of the noise and the lymphocytes was improved, and the separation accuracy of the monocytes and the granulocytes was improved.

[Table 6]

| Additive reagent | Pulse (p) range in each area Pulse number Proportion | | | Total pulse number |
|---|---|---|---|---|
| | Area 2 (lymphocyte) | Area 3 (monocyte) | Area 4 (granulocyte) | |
| Comparative Example 3-1 none (basic reagent) | $975 \leq p < 2000$ 3735 28.80% | $2000 \leq p < 3200$ 2160 54.50% | $3200 \leq p < 9925$ 7061 54.50% | 12956 |
| Comparative Example 3-2 lauryltrimethylammonium chloride | $900 \leq p < 2100$ 4496 7.50% | $2100 \leq p < 2850$ 1205 10.10% | $2850 \leq p < 9925$ 6288 52.40% | 11989 |
| Comparative Example 3-3 polyoxyethylene lauryl ether | $1250 \leq p < 3175$ 4804 40.60% | $3175 \leq p < 3875$ 831 7.00% | $3875 \leq p < 9925$ 6204 52.40% | 11839 |
| Example 3-1 sucrose laurate | $1100 \leq p < 2750$ 5121 43.60% | $2750 \leq p < 3450$ 688 5.90% | $3450 \leq p < 9925$ 5935 50.50% | 11744 |
| Example 3-2 sucrose monolaurate | $1025 \leq p < 2400$ 4559 40.20% | $2400 \leq p < 3275$ 1063 9.40% | $3275 \leq p < 9925$ 5706 50.40% | 11328 |
| Example 3-3 sucrose monocaprate | $1000 \leq p < 2400$ 4498 39.80% | $2400 \leq p < 3275$ 1248 11.00% | $3275 \leq p < 9925$ 5563 49.20% | 11309 |
| Example 3-4 dodecyl maltoside | $1000 \leq p < 2175$ 4658 40.00% | $2175 \leq p < 2775$ 885 7.60% | $2775 \leq p < 9925$ 6112 52.40% | 11655 |

(Example 4)

In Example 4, the leukocytes were measured in the same manner as in Example 3 except that the final concentration of the lauryl trimethyl ammonium chloride in the basic reagent was 12.3 mmol/L, and the respective analysis reagents obtained by adding sucrose monolaurate to the basic reagent shown in Table 5 were used. The concentration of the sucrose laurate added to the analysis reagent in Example 4 will be shown in Table 7 below. In Table 7, the final concentration is a concentration of the sucrose laurate in the mixed solution obtained by mixing the analysis reagent of Example 4 and a sample.

(Table 7)

| | sucrose laurate | |
|---|---|---|
| Examples | Added concentration (w/v%) | Final concentration (w/v%) |
| Comparative Example 4 (basic reagent) | 0 (additive reagent-free) | 0 (additive reagent-free) |
| Example 4-1 | 0.0125 | 0.0038 |
| Example 4-2 | 0.025 | 0.0075 |
| Example 4-3 | 0.05 | 0.015 |
| Example 4-4 | 0.1 | 0.03 |
| Example 4-5 | 0.2 | 0.06 |

In FIG. 11 and Table 8 below, the analysis results of the leukocytes in Comparative Example 4 and Examples 4-1 to 4-5 are shown. FIGs. 11A to 11F are histograms showing particle size distributions of leukocytes in the analyses using the analysis reagents to which sucrose laurate was added so that their concentration becomes the predetermined concentrations. FIG. 11A is a histogram showing a result of analysis using the analysis reagent of Comparative Example 4 (basic reagent). FIG. 11B is a histogram showing a result of analysis using the analysis reagent of Example 4-1. FIG. 11C is a histogram showing a result of analysis using the analysis reagent of Example 4-2. FIG. 11D is a histogram showing a result of analysis using the analysis reagent of Example 4-3. FIG. 11E is a histogram showing a result of analysis using the analysis reagent of Example 4-4. FIG. 11F is a histogram showing a result of analysis using the analysis reagent of Example 4-5. In the histograms shown in FIGs. 11A to 11F, the horizontal axis represents the pulse height, while the vertical axis represents the pulse number. In the histograms, the leukocytes with the pulse heights in area 2 were classified as the small size (lymphocytes), the leukocytes with the pulse heights in area 3 were classified as the middle size (monocytes), and the leukocytes with the pulse heights in area 4 were classified as the large size (granulocytes). It is to be noted that the area 1 denotes a blood component (noise) other than the leukocytes. The following Table 8 shows, with respect to the respective areas, the pulse ranges, pulse numbers, and their proportions obtained by the respective analyses using the respective analysis reagents.

As shown in FIG. 11A, in the case where the basic reagent of Comparative Example 4 was used, the valley between the area 1 (noise) and the area 2 (lymphocytes) became shallow, and the separation accuracy of the area 1 (noise) and the area 2 (lymphocytes) became low. On the other hand, as shown in FIGs. 11B to 11F, in the case where the analysis reagent containing sucrose laurate was used, the height up to the peak of the valley between the area 1 (noise) and the area 2 (lymphocytes) became low, and the separation accuracy of the noise and the lymphocytes was improved. Among them, in the case where the final concentration of sucrose laurate in the mixed solution was in the range of 0.0075 to 0.06 w/v% (FIGs. 11C to 11F), the separation accuracy became higher, and in the case where the final concentration was 0.03% (FIG. 11E), the separation accuracy was the highest.

[Table 8]

| Analysis reagent (w/v%)* | Pulse (p) range in each area Pulse number Proportion | | | Total pulse number |
|---|---|---|---|---|
| | Area 2 (lymphocyte) | Area 3 (monocyte) | Area 4 (granulocyte) | |
| Comparative Example 4-1 (0) | $1275 \leq p < 3500$ 2015 33.40% | $3500 \leq p < 4225$ 319 5.30% | $4225 \leq p < 9925$ 3698 61.30% | 6032 |
| Example 4-1 (0.0038) | $1250 \leq p < 2500$ 1200 24.60% | $2500 \leq p < 3200$ 452 9.20% | $3200 \leq p < 9925$ 3235 66.20% | 4887 |
| Example 4-2 (0.0075) | $1250 \leq p < 2350$ 1240 22.80% | $2350 \leq p < 3100$ 595 10.90% | $3100 \leq p < 9925$ 3604 66.30% | 5439 |
| Example 4-3 (0.015) | $1575 \leq p < 3300$ 1392 23.60% | $3300 \leq p < 3825$ 349 5.90% | $3825 \leq p < 9925$ 4169 70.50% | 5910 |
| Example 4-4 (0.03) | $1325 \leq p < 3250$ 2268 35.00% | $3250 \leq p < 4000$ 453 7.00% | $4000 \leq p < 9925$ 3758 58.00% | 6479 |
| Example 4-5 (0.06) | $1475 \leq p < 3300$ 2194 35.80% | $3300 \leq p < 3825$ 490 8.00% | $3825 \leq p < 9925$ 3437 56.20% | 6121 |
| * final concentration of sucrose laurate in a mixed solution | | | | |

As above, by the use of the analysis reagent of the present invention containing a nonionic surfactant that contains a sugar residue as a hydrophilic region and an aliphatic chain as a hydrophobic region, it is possible to carry out an stable analysis of leukocytes even in the case where, for example, a dilution ratio of a sample is low, and a flow velocity of the sample during the measurement is slow. Further, by the use of the analysis reagent of the present invention, the measurement with high accuracy can be achieved even in the case mentioned above, for example.

Industrial Applicability

According to the analysis method of the present invention, leukocytes can be analyzed stably under the condition where a dilution ratio is low and also under the condition where a flow velocity is slow, as in the case of using a μTAS or the like. The present invention can be applied to all fields in which leukocytes are analyzed, such as clinical examinations, biochemical examinations, and medical researches. The use of the present invention is not limited, and the present invention can be applied to a wide range of fields.

Reference Signs List

| | |
|---|---|
| 1 | cartridge |
| 2 | upper substrate |
| 3 | lower substrate |
| 4 | sample metering part |
| 41 | sample introducing part |
| 42 | sample introducing flow path |
| 43 | branch part |
| 44 | overflow channel |
| 45, 55, 58, 65 | drain |
| 46 | sample metering flow path |
| 47 | orifice |
| 5 | diluting part |
| 51 | reagent bath |
| 52 | regent introducing flow path |
| 53 | branch part |
| 54 | overflow channel |
| 56 | reagent metering flow path |
| 57 | tapering part |
| 59 | diluting bath |
| 6 | analyzing part |
| 61, 62 | electrode, electrode arranging part |
| 14, 63, 84 | fine through-hole |
| 64 | flow rate measuring flow path |
| 7 | connector |
| 8, 88 | leukocyte analysis device |
| 9 | main bath |
| 10 | sub bath |
| 11 | sucking part |
| 12, 13 | electrode |
| 15 | quantitative determination part |
| 16 | substrate part |
| 17 | stirring bath |
| 18 | stirring pipe |
| 19 | sucking pipe |
| 21a, 21b | through hole, inlet |
| 22a, 22b, 22c, 22d, 22e | through hole |
| 90 | main bath part |
| 100 | sub bath part |
| 160 | stirring part |
| 91a, 91b, 101a, 101b, 161a, 161b | substrate |
| 840 | elastic body |

**Claims**

**1.** A method for analyzing a leukocyte, comprising the steps of

mixing a sample containing a leukocyte and an erythrocyte and an analysis reagent containing a surfactant that reacts with leukocytes; and

measuring the leukocyte by passing a mixed solution of the sample and the analysis reagent through a fine through-hole, measuring a signal detected when the mixed solution passes through the fine through-hole, and classifying and counting the leukocyte in the sample, wherein

the analysis reagent further contains a nonionic surfactant, and

the nonionic surfactant has a sugar residue as a hydrophilic region and an aliphatic chain as a hydrophobic region.

2. The method according to claim 1, wherein the sugar residue is a disaccharide residue.

3. The method according to claim 1, wherein the aliphatic chain is a fatty acid residue or an alkyl group.

4. The method according to claim 1, wherein the nonionic surfactant is at least one selected from the group consisting of sucrose monolaurate, sucrose laurate, sucrose monocaprate, and dodecyl maltoside.

5. The method according to clam 1, wherein a concentration of the nonionic surfactant in the mixed solution of the sample and the analysis reagent is in a range of 0.001 to 5 w/v%.

6. The method according to claim 1, wherein the surfactant that reacts with leukocytes is a quaternary ammonium salt.

7. The method according to claim 1, wherein a concentration of the surfactant that reacts with leukocytes in the mixed solution of the sample and the analysis reagent is in a range of 0.01 to 5 w/v%.

8. The method according to claim 1, wherein the analysis reagent further comprises a surfactant that reacts with erythrocytes.

9. The method according to claim 8, wherein the surfactant that reacts with erythrocytes is a saponin.

10. The method according to claim 9, wherein a concentration of the saponin in the mixed solution of the sample and the analysis reagent is in a range of 0.05 to 5 w/v%.

11. The method according to claim 1, wherein in the mixing step, a mixed volume ratio (X : Y) of the sample (X) to the analysis reagent (Y) is in a range of 1 : 0.4 to 1 : 99.

12. The method according to claim 1, wherein in the measuring step, the leukocyte is classified into three types according to its volumes.

13. The method according to claim 1, wherein in the measuring step,
a cartridge having the fine through-hole is used, and
the mixed solution is passed through the fine through-hole in the cartridge.

14. The method according to claim 13, wherein the cartridge contains a micro total analysis system.

15. An analysis reagent used for the analysis method according to claim 1, comprising a surfactant that reacts with leukocytes, wherein
the analysis reagent further contains a nonionic surfactant, and
the nonionic surfactant has a sugar residue as a hydrophilic region and an aliphatic chain as a hydrophobic region.

16. The analysis reagent according to claim 15, wherein the sugar residue is a disaccharide.

17. The analysis reagent according to claim 15, wherein the aliphatic chain is a fatty acid residue or an alkyl group.

18. The analysis reagent according to claim 15, wherein the nonionic surfactant is at least one selected from the group consisting of sucrose monolaurate, sucrose laurate, sucrose monocaprate, and dodecyl maltoside.

19. The analysis reagent according to claim 15, wherein the surfactant that reacts with leukocytes is a quaternary ammonium salt.

**20.** The analysis reagent according to claim 15, further containing a surfactant that reacts with erythrocytes.

**21.** The analysis reagent according to claim 20, wherein the surfactant that reacts with erythrocytes is a saponin.

FIG. 1A

FIG. 1B

FIG. 2

FIG. 3A

FIG. 3B

FIG. 3C

FIG. 4A

FIG. 4B

FIG. 4C

FIG. 5A

FIG. 5B

FIG. 5C

FIG. 6A

FIG. 6B

FIG. 6C

88

11
19
22d
17
101a    101b
21a
15
91a    9    91b
22a
12
22c
161a    161b
22e
18
16
160
10
22b
13
14
90
21b
100

FIG. 7

FIG. 8

FIG. 9A

FIG. 9B

FIG. 9C

area

FIG. 10A

area

FIG. 10B

area

FIG. 10C

area

FIG. 10D

FIG. 11A

FIG. 11B

FIG. 11C

FIG. 11D

FIG. 11E

FIG. 11F

## INTERNATIONAL SEARCH REPORT

| | International application No. |
|---|---|
| | PCT/JP2009/058489 |

A. CLASSIFICATION OF SUBJECT MATTER
*G01N33/49*(2006.01)i

According to International Patent Classification (IPC) or to both national classification and IPC

B. FIELDS SEARCHED

Minimum documentation searched (classification system followed by classification symbols)
G01N33/49

Documentation searched other than minimum documentation to the extent that such documents are included in the fields searched

| Jitsuyo Shinan Koho | 1922-1996 | Jitsuyo Shinan Toroku Koho | 1996-2009 |
|---|---|---|---|
| Kokai Jitsuyo Shinan Koho | 1971-2009 | Toroku Jitsuyo Shinan Koho | 1994-2009 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X/Y | JP 06-273413 A  (Toa Medical Electronics Co., Ltd.), 30 September, 1994 (30.09.94), Claims; Par. Nos. [0005], [0031] & US 5413938 A        & EP 617281 A3 & DE 69419053 C        & ES 2132352 T & CA 2119390 A        & CN 1095481 A | 1-5,15-18/ 6-14,19-21 |
| Y | JP 10-160730 A  (Abbot Laboratories), 19 June, 1998 (19.06.98), Par. No. [0071] & US 5656499 A        & US 5631165 A & WO 1996/004542 A1     & DE 69527292 D & AU 3071895 A        & CA 2193510 A & ES 2180650 T | 6-14,19-21 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 July, 2009 (08.07.09) | 21 July, 2009 (21.07.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2009/058489

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2002-277380 A  (Horiba, Ltd.),<br>25 September, 2002 (25.09.02),<br>Claims; Par. No. [0002]; Figs. 1, 3<br>(Family: none) | 12-14 |
| A | JP 61-502277 A  (Coulter Electronics Inc.),<br>09 October, 1986 (09.10.86),<br>& US 4751179 A        & EP 185048 A<br>& WO 1985/005684 A1      & DE 3587036 A | 1-21 |
| A | JP 2003-329668 A  (Sysmex Corp.),<br>19 November, 2003 (19.11.03),<br>& US 2003/0219850 A1 | 1-21 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3143064 B